(19) Europäisches Patentamt
European Patent Office
Office européen des brevets

(11) Veröffentlichungsnummer: **0 317 845 A2**

# (12) EUROPÄISCHE PATENTANMELDUNG

(21) Anmeldenummer: **88118817.1**

(22) Anmeldetag: **11.11.88**

(51) Int. Cl.⁴: **C07D 207/323 , C07D 307/52 , C07D 333/20 , A61K 31/40 , A61K 31/34 , A61K 31/38 , C07C 83/08 , A61K 31/215**

(30) Priorität: **25.11.87 DE 3739882**

(43) Veröffentlichungstag der Anmeldung: **31.05.89 Patentblatt 89/22**

(84) Benannte Vertragsstaaten: **AT BE CH DE ES FR GB GR IT LI NL SE**

(71) Anmelder: **BAYER AG**

**D-5090 Leverkusen 1 Bayerwerk(DE)**

(72) Erfinder: **Hübsch, Walter, Dr.**
**Am Eckbush 43/50**
**D-5600 Wuppertal 1(DE)**
Erfinder: **Angerbauer, Rolf, Dr.**
**Sterntalerweg 29**
**D-5600 Wuppertal 1(DE)**
Erfinder: **Fey, Peter, Dr.**
**Ursulastrasse 14**
**D-5600 Wuppertal 1(DE)**
Erfinder: **Bischoff, Hilmar, Dr.**
**Am Rohm 78**
**D-5600 Wuppertal 1(DE)**
Erfinder: **Petzinna, Dieter, Dr.**
**Peter-Berten-Strasse 10**
**D-4000 Düsseldorf 12(DE)**
Erfinder: **Schmidt, Delf, Dr.**
**Am Eckbusch 55b**
**D-5600 Wuppertal 11(DE)**

## (54) Substituierte Hydroxylamine.

(57) Neue substituierte Hydroxylamine können durch Umsetzung von entsprechenden monosubstituierten Hydroxylaminen mit Epoxyden hergestellt werden. Die neuen substituierten Hydroxylamine können in Arzneimitteln verwendet werden.

EP 0 317 845 A2

## Substituierte Hydroxylamine

Die Erfindung betrifft substituierte Hydroxylamine, Zwischenverbindungen zu ihrer Herstellung, ihre Herstellung und ihre Verwendung in Arzneimitteln.

Es ist bekannt, daß aus Pilzkulturen isolierte Lactonderivate Inhibitoren der 3-Hydroxy-3-methyl-glutaryl Coenzym A Reduktase (HMG-CoA-Reduktase) sind [Mevinolin, EP-A 22 478; US 4 231 938]. Darüberhinaus sind auch bestimmte Indolderivate bzw. Pyrazolderivate Inhibitoren der HMG-CoA-Reduktase [EP-A 1 114 027; US 4 613 610].

Es wurden nun substituierte Hydroxylamine der allgemeinen Formel (I),

$$R^1\text{-}CH_2CH_2\text{-}\overset{OH}{\overset{|}{N}}\text{-}CH_2\text{-}\overset{OH}{\overset{|}{C}}H\text{-}CH_2\text{-}COOR \qquad (I)$$

in welcher

R - für Wasserstoff,

- für einen Esterrest, oder

- für ein Kation steht,

und

$R^1$ - für eine Gruppe der Formel

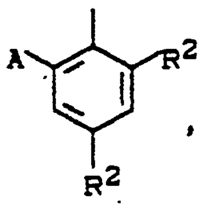 , 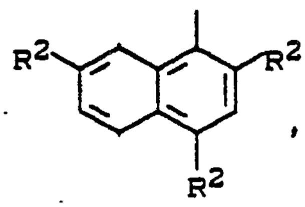 , 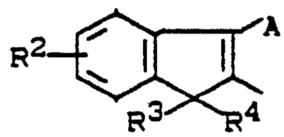 ,

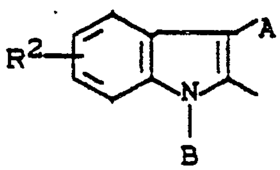 , 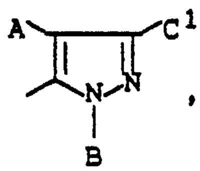 , 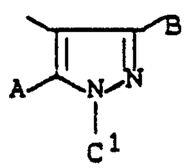 ,

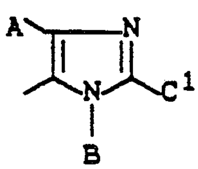 , 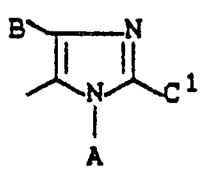 , 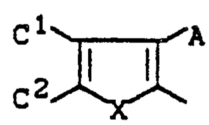 ,

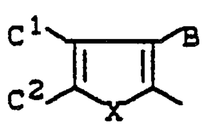 , 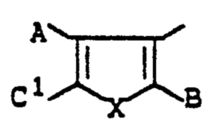 oder 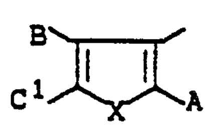

steht,

worin

$R^2$ - Wasserstoff, Halogen oder geradkettiges oder verzweigtes Alkyl mit bis zu 6 Kohlenstoffatomen bedeutet,

$R^3$, $R^4$ - gleich oder verschieden sind und

- Wasserstoff, Halogen oder geradkettiges oder verzweigtes Alkyl mit bis zu 6 Kohlenstoffatomen bedeuten, oder

$R^3$ und $R^4$ gemeinsam eine Tetramethylenkette bilden,

A - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio; Alkoxycarbonyl oder durch eine Gruppe der Formel $-NR^5R^6$ substituiert sein kann,
worin
$R^5$, $R^6$ - gleich oder verschieden sind und Alkyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
B - Cycloalkyl bedeutet, oder
- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
X - O, S oder $N-C^3$ bedeutet,
und
$C^1$, $C^2$, $C^3$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ die oben angegebene Bedeutung haben,
- oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste bis zu 3-fach gleich oder verschieden durch Halogen, Cyano, Tri fluormethyl, Trifluormethoxy, Alkyl, Alkoxy, Alkylthio oder Alkylsulfonyl substituiert sein kann,
oder
- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,
worin
$R^5$ und $R^6$ die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
gefunden.

Überraschenderweise zeigen die erfindungsgemäßen substituierten Hydroxylamine eine überlegene inhibitorische Wirkung auf die HMG-CoA Reduktase (3-Hydroxy-3-methylglutaryl Coenzym A Reduktase).

Cycloalkyl steht im allgemeinem für einen cyclischen Kohlenwasserstoffrest mit 3 bis 8 Kohlenstoffatomen. Bevorzugt ist der Cyclopropyl-, Cyclopentan- und der Cyclohexanring. Beispielsweise seien Cyclopropyl, Cyclopentyl, Cyclohexyl, Cycloheptyl und Cyclooctyl genannt.

Alkyl steht im allgemeinen für einen verzweigten Kohlenwasserstoffrest mit 1 bis 12 Kohlenstoffatomen.

Bevorzugt wird Niederalkyl mit 1 bis etwa 6 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl genannt.

Alkoxy steht im allgemeinen für einen über ein Sauerstoffatom gebundenen geradkettigen oder verzweigten Kohlenwasserstoffrest mit 1 bis 12 Kohlenstoffatomen. Bevorzugt ist Niederalkoxy mit 1 bis etwa 6 Kohlenstoff atomen. Besonders bevorzugt ist ein Alkoxyrest mit 1 bis 4 Kohlenstoffatomen. Beispielsweise seien Methoxy, Ethoxy, Propoxy, Isopropoxy, Butoxy, Isobutoxy, Pentoxy, Isopentoxy, Hexoxy, Isohexoxy, Heptoxy, Isoheptoxy, Octoxy oder Isooctoxy genannt.

Alkylthio steht im allgemeinen für einen über ein Schwefelatom gebundenen geradkettigen oder verzweigten Kohlenwasserstoffrest mit 1 bis 12 Kohlenstoffatomen. Bevorzugt ist Niederalkylthio mit 1 bis etwa 6 Kohlenstoffatomen. Besonders bevorzugt ist ein Alkylthiorest mit 1 bis 4 Kohlenstoffatomen. Beispielsweise seien Methylthio, Ethylthio, Propylthio, Isopropylthio, Butylthio, Isobutylthio, Pentylthio, Isopentylthio, Hexylthio, Isohexylthio, Heptylthio, Isoheptylthio, Octylthio oder Isooctylthio genannt.

Alkylsulfonyl steht im allgemeinen für einen geradkettigen oder verzweigten Kohlenwasserstoffrest mit 1 bis 12 Kohlenstoffatomen, der über eine $SO_2$-Gruppe gebunden ist. Bevorzugt ist Niedrigalkylsulfonyl mit 1 bis etwa 6 Kohlenstoffatomen. Beispielsweise seien genannt: Methylsulfonyl, Ethylsulfonyl, Propylsulfonyl, Isopropylsulfonyl, Butylsulfonyl, Isobutylsulfonyl, Pentylsulfonyl, Isopentylsulfonyl, Hexylsulfonyl, Isohexylsulfonyl.

Sulfamoyl (Aminosulfonyl) steht für die Gruppe $-SO_2-NH_2$.

Aryl steht im allgemeinen für einen aromatischen Rest mit 6 bis etwa 12 Kohlenstoffatomen. Bevorzugte Arylreste sind Phenyl, Naphthyl und Biphenyl.

Aryloxy steht im allgemeinen für einen aromatischen Rest mit 6 bis etwa 12 Kohlenstoffatomen, der über ein Sauerstoffatom gebunden ist. Bevorzugte Aryloxyreste sind Phenoxy oder Naphthyloxy.

Arylthio steht im allgemeinen für einen aromatischen Rest mit 6 bis etwa 12 Kohlenstoffatomen, der über ein Schwefelatom gebunden ist. Bevorzugte Arylthioreste sind Phenylthio oder Naphthylthio.

Arylsulfonyl steht im allgemeinen für einen aromatischen Rest mit 6 bis etwa 12 Kohlenstoffatomen, der über eine $SO_2$-Gruppe gebunden ist. Beispielsweise seien genannt: Phenylsulfonyl, Naphthylsulfonyl und Biphenylsulfonyl.

Aralkyl steht im allgemeinen für einen über eine Alkylenkette gebundenen Arylrest mit 7 bis 14 Kohlenstoffatomen. Bevorzugt werden Aralkylreste mit 1 bis 6 Kohlenstoffatomen im aliphatischen Teil und 6 bis 12 Kohlenstoffatome im aromatischen Teil. Beispielsweise seien folgende Aralkylreste genannt: Benzyl, Naphthylmethyl, Phenethyl und Phenylpropyl.

Aralkoxy steht im allgemeinen für einen Aralkylrest mit 7 bis 14 Kohlenstoffatomen, wobei die Alkylenkette über ein Sauerstoffatom gebunden ist. Bevorzugt werden Aralkoxyreste mit 1 bis 6 Kohlenstoffatomen im aliphatischen Teil und 6 bis 12 Kohlenstoffatomen im aromatischen Teil. Beispielsweise seien folgende Aralkoxyreste genannt: Benzyloxy, Naphthylmethoxy, Phenethoxy und Phenylpropoxy.

Aralkylthio steht im allgemeinen für einen Aralkylrest mit 7 bis etwa 14 Kohlenstoffatomen wobei die Alkylkette über ein Schwefelatom gebunden ist. Bevorzugt werden Aralkylthioreste mit 1 bis 6 Kohlenstoffatomen im aliphatischen Teil und 6 bis 12 Kohlenstoffatomen im aromatischen Teil. Beispielsweise seien folgende Aralkylthioreste genannt: Benzylthio, Naphthylmethylthio, Phenethylthio und Phenylpropylthio.

Aralkylsulfonyl steht im allgemeinen für einen Aralkylrest mit 7 bis etwa 14 Kohlenstoffatomen, wobei die Alkylreste über eine $SO_2$-Kette gebunden ist. Bevorzugt werden Aralkylsulfonylreste mit 1 bis 6 Kohlenstoffatomen im aliphatischen Teil und 6 bis 12 Kohlenstoffatomen im aromatischen Teil. Beispielsweise seien folgende Aralkylsulfonylreste genannt: Benzylsulfonyl, Naphthylmethylsulfonyl, Phenethylsulfonyl und Phenylpropylsulfonyl.

Alkoxycarbonyl kann beispielsweise durch die Formel

$$-\underset{\underset{O}{\|}}{C}-OAlkyl$$

dargestellt werden. Alkyl steht hierbei für einen geradkettigen oder verzweigten Kohlenwasserstoffrest mit 1 bis 12 Kohlenstoffatomen. Bevorzugt wird Niederalkoxycarbonyl mit 1 bis etwa 6 Kohlenstoffatomen im Alkylteil. Insbesondere bevorzugt wird ein Alkoxycarbonyl mit 1 bis 4 Kohlenstoffatomen im Alkylteil. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.

Acyl steht im allgemeinen für Phenyl oder geradkettiges oder verzweigtes Niedrigalkyl mit 1 bis etwa 6 Kohlenstoffatomen, die über eine Carbonylgruppe gebunden sind. Bevorzugt sind Phenyl und Alkylreste mit bis zu 4 Kohlenstoffatomen. Beispielsweise seien genannt: Benzoyl, Acetyl, Ethylcarbonyl, Propylcarbonyl, Isopropylcarbonyl, Butylcarbonyl und Isobutylcarbonyl.

Halogen steht im allgemeinen für Fluor, Chlor, Brom oder Iod, bevorzugt für Fluor, Chlor oder Brom. Besonders bevorzugt steht Halogen für Fluor oder Chlor.

Heteroaryl steht im allgemeinen für einen 5- bis 6-gliedrigen aromatischen Ring, der als Heteroatome Sauerstoff, Schwefel und/oder Stickstoff enthalten kann und an den weitere aromatische Ringe ankondensiert sein können. Bevorzugt sind 5- und 6-gliedrige aromatische Ringe, die einen Sauerstoff, ein Schwefel und/oder bis zu 2 Stickstoffatome enthalten und die gegebenenfalls benzokondensiert sind. Als besonders bevorzugte Heteroarylreste seien genannt: Thienyl, Furyl, Pyrolyl, Pyrazolyl, Pyridyl, Pyrimidyl, Pyrazinyl, Pyridazinyl, Chinolyl, Isochinolyl, Chinazolyl, Chinoxalyl, Phthalazinyl, Cinnolyl, Thiazolyl, Benzothiazolyl, Isothiazolyl, Oxazolyl, Benzoxazolyl, Isoxazolyl, Imidazolyl, Benzimidazolyl, Pyrazolyl, Indolyl, und Isoindolyl.

Steht $R^1$ für einen Esterrest so ist hiermit bevorzugt ein physiologisch verträglicher Esterrest gemeint, der in vivo leicht zu einer freien Carboxylgruppe und einem entsprechenden physiologisch verträglichen Alkohol hydrolysiert wird. Hierzu gehören beispielsweise Alkylester ($C_1$ bis $C_4$) und Aralkylester ($C_7$ bis $C_{10}$), bevorzugt Niederalkylester sowie Benzylester. Darüber hinaus seien die folgenden Esterreste genannt: Methylester, Ethylester, Propylester, Benzylester.

Steht $R^1$ für ein Kation so, ist bevorzugt ein physiologisch verträgliches Metall-oder Ammoniumkation gemeint. Bevorzugt sind hierbei Alkali-bzw. Erdalkalikationen wie beispielsweise Natrium-, Kalium-, Magnesium- oder Calciumkationen, sowie Aluminium-oder Ammoniumkationen, sowie nicht-toxische substituierte Ammoniumkationen aus Aminen wie Diniedrigalkylamine, Triniedrigalkylamine, Prokain, Dibenzylamin, N,N′-Dibenzylethylendiamin, N-Benzyl-β-phenylethylamin, N-Methylmorpholin oder N-Ethylmorpholin, 1-Ephenamin, Dihydroabiethylamin, N,N′-Bis-dihydroabiethylethylendiamin, N-Niedrigalkylpiperidin und andere Amine, die zur Bildung von Salzen verwendet werden können.

Bevorzugt sind solche Verbindungen der allgemeinen Formel (I),

in welcher

R - für Wasserstoff oder

- für einen physiologisch verträglichen Esterrest steht, oder

- für ein physiologisch verträgliches Kation steht,

und

$R^1$ - für eine Gruppe der Formel

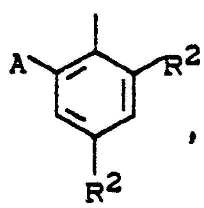 , 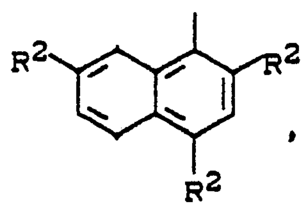 , 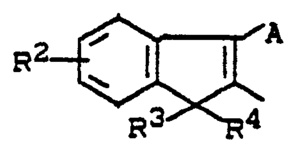 ,

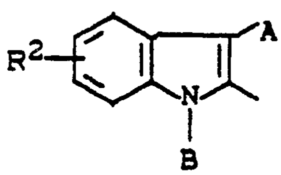 , 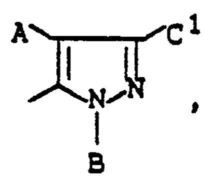 , 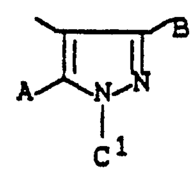 ,

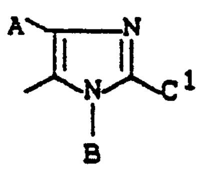 , 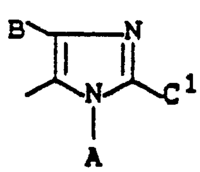 , 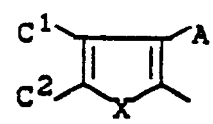 ,

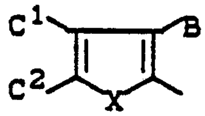 , 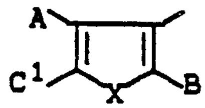 oder 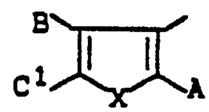

steht,

worin

$R^2$ - Wasserstoff, Fluor, Chlor, Brom, oder

- geradkettiges oder verzweigtes Alkyl mit bis zu 5 Kohlenstoffatomen bedeutet,
$R^3$, $R^4$ - gleich oder verschieden sind und
- Wasserstoff, Fluor, Chlor, Brom, oder
- geradkettiges oder verzweigtes Alkyl mit bis zu 4 Kohlenstoffatomen bedeuten,
oder
$R^3$ und $R^4$ gemeinsam eine Tetramethylenkette bilden,
A - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ gleich oder verschieden sind und Niederalkyl, Phenyl, Benzyl, Acetyl, Benzoyl, Phenylsulfonyl oder Niederalkylsulfonyl bedeuten,
B - Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ die oben angegebene Bedeutung haben,
oder durch Pyridyl, Pyrimidyl, Pyrazinyl, Pyridazinyl, Chinolyl, Isochinolyl, Pyrryl, Indolyl, Thienyl, furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,
X - O, S oder $N-C^3$ bedeutet,
und
$C^1$, $C^2$, $C^3$ gleich oder verschieden sind und
- Wasserstoff, oder
- Cyclopropyl, Cyclopentyl oder Cyclohexyl bedeuten, oder
- Niederalkyl bedeuten, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ die oben angegebene Bedeutung haben,
oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Bro, Niederalkyl, Niederalkoxy, Trifluormethyl, oder Trifluormethoxy substituiert sein können,
- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ die oben angegebene Bedeutung haben.

Besonders bevorzugt sind Verbindungen der allgemeinen Formeln (I),

in welcher
R - für Wasserstoff, Methyl, Ethyl, Propyl, Isopropyl, Butyl, Isobutyl, tert.Butyl oder Benzyl, oder
- für ein Magnesium- oder Ammoniumkation steht,
und
$R^1$ - für eine Gruppe der Formel

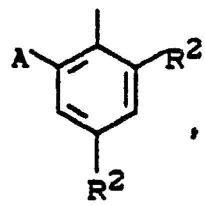 , 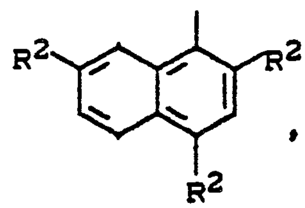 , 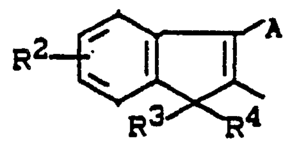 ,

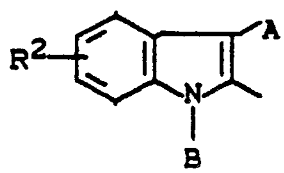 , 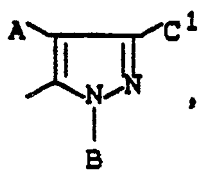 , 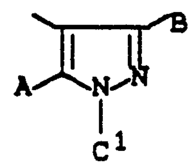 ,

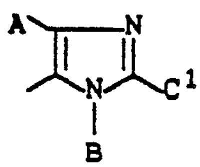 , 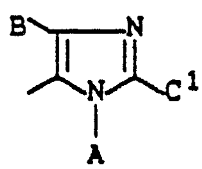 , 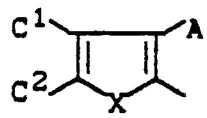 ,

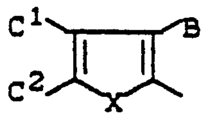 , 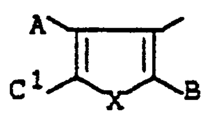 oder 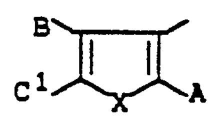

steht,
worin
$R^2$ - Wasserstoff, Fluor, Chlor oder
- geradkettiges oder verzweigtes Alkyl mit bis zu 4 Kohlenstoffatomen bedeutet,
$R^3$, $R^4$ - gleichzeitig Wasserstoff, Fluor, Chlor oder
- Alkyl mit bis zu 3 Kohlenstoffatomen bedeuten,
oder
$R^3$ und $R^4$ gemeinsam eine Tetramethylenkette bilden,
A - Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
B - Cyclopropyl, Cyclopentyl oder Cyclohexyl bedeutet, oder
- Methyl, Ethyl, Propyl, Isopropyl, Butyl, sec.Butyl oder tert.Butyl bedeutet, das substi tuiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
X - O, S oder N-$C^3$ bedeutet,

$C^1$, $C^2$, $R^3$ gleich oder verschieden sind und
- Wasserstoff, Cyclopropyl, Cyclopentyl oder Cyclohexyl bedeuten, oder
- Methyl, Ethyl, Propyl, Isopropyl, Butyl, Isobutyl, tert.Butyl, Pentyl, Isopentyl, Hexyl oder Isohexyl bedeuten, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^5R^6$,
wobei
$R^5$ und $R^6$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
- oder durch Pyridyl, Pyrimidyl, Pyrazinyl, Pyridazinyl, Chinolyl, Isochinolyl, Thienyl, furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^5R^6$ substituiert sein kann,
wobei
$R^5$ und $R^6$ die oben angegebene Bedeutung haben.

Ganz besonders bevorzugt sind Verbindung der allgemeinen Formel (I),
in welcher
R - für Wasserstoff, Methyl oder Ethyl steht, oder
- für ein Natrium- oder Kaliumkation steht,
und
$R^1$ - für eine Gruppe der Formel

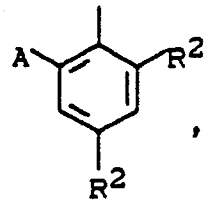, 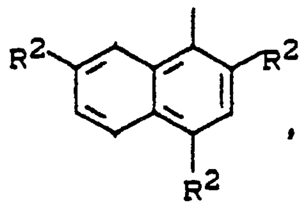, 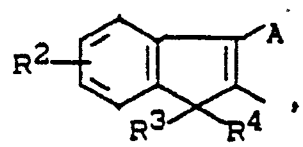,

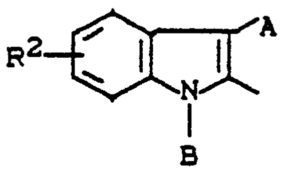, 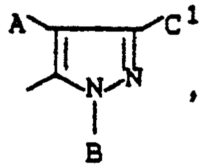, 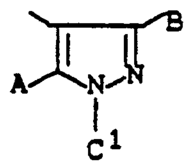,

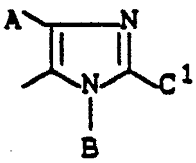, 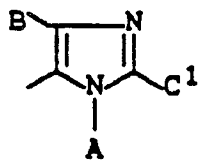, 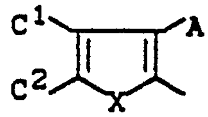,

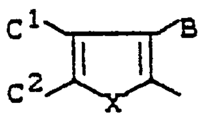, 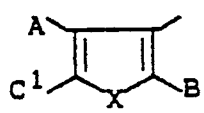 oder 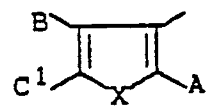

steht,
worin
$R^2$ - Wasserstoff, Fluor, Chlor oder
- geradkettiges oder verzweigtes Alkyl mit bis zu 4 Kohlenstoffatomen bedeutet,
$R^3$, $R^4$ - gleichzeitig Wasserstoff und Methyl, oder
- Alkyl mit bis zu 3 Kohlenstoffatomen bedeuten,
oder
$R^3$ und $R^4$ gemeinsam eine Tetramethylenkette bilden,
A - Phenyl bedeutet, das bis zu 2-fach gleich oder verschieden durch Methyl, Ethyl, Propyl, Isopropyl, Butyl, Isobutyl, tert.Butyl, Methoxy, Ethoxy, Propoxy, Isopropoxy, Phenoxy, Benzyloxy, Fluor, Chlor, Brom, Cyano, Trifluormethyl oder Trifluormethoxy substituiert sein kann,
B - Cyclopropyl, Methyl, Ethyl, Propyl, Isopropyl, Butyl, Isobutyl oder tert.Butyl bedeutet, das durch Fluor, Chlor, Methoxy, Phenyl oder Phenoxy substituiert sein kann,
X - O, S oder N-$C^3$ bedeutet,
$C^1$, $C^2$, $C^3$ gleich oder verschieden sind und
- Wasserstoff, Cyclopropyl, Cyclopentyl oder Cyclohexyl bedeuten, oder
- Methyl, Ethyl, Propyl, Isopropyl, Butyl, Isobutyl, tert.Butyl, Pentyl, Isopentyl, Hexyl oder Isohexyl bedeuten, das substituiert sein kann durch Fluor, Chlor, Methoxy, Ethoxy, Propoxy, Isopropoxy, Methylthio, Ethylthio, Methylsulfonyl, Ethylsulfonyl, Ethoxycarbonyl, Propoxycarbonyl, Isopropoxycarbonyl oder durch ein Gruppe der Formel $NR^5R^6$,
wobei
$R^5$ und $R^6$ gleich oder verschieden sind und
- für Wasserstoff, Methyl, Ethyl, Propyl, Isopropyl, Phenyl oder Benzyl stehen,
oder durch gegebenenfalls durch Fluor, Chlor, Methyl, Methoxy, Trifluormethyl oder Trifluormethoxy substituiertes Pyridyl, Pyrimidyl, Chinolyl, Thienyl, Furyl, Phenyl, Phenoxy, Phenylsulfonyl oder Benzyloxy,
oder
- gegebenenfalls durch Fluor, Chlor, Methyl, Ethyl, Propyl, Isopropyl, Methoxy, Phenyl, Methoxycarbonyl, Ethoxycarbonyl substituiertes Thienyl, Furyl, Pyridyl, Pyrimidyl, Chinolyl, Isochinolyl, Benzoxazolyl, Benzthiazolyl oder Benzimidazolyl bedeutet, oder

- Phenyl bedeutet, das bis zu 2-fach gleich oder verschieden durch Methyl, Ethyl, Propyl, Isopropyl, Butyl, Isobutyl, tert.Butoxy, Methoxy, Ethoxy, Propoxy, Isopropoxy, Methylthio, Ethylthio, Methylsulfonyl, Ethylsulfonyl, Phenyl, Phenoxy, Phenylsulfonyl, Benzyloxy, Fluor, Chlor, Brom, Cyano, Trifluormethyl, Trifluormethoxy, Methoxycarbonyl, Ethoxycarbonyl oder eine Gruppe der Formel $-NR^5R^6$ substituiert sein kann, wobei
$R^5$ und $R^6$ die oben angegebene Bedeutung haben.

Die erfindungsgemäßen substituierten Hydroxylamine der allgemeinen Formel (I) haben mindestens ein asymmetrisches Kohlenstoffatom (Kohlenstoffatom 3) und können daher in verschiedenen stereochemischen Formen existieren. Sowohl die einzelnen Isomeren als auch deren Mischungen sind Gegenstand der Erfindung.

Beispielsweise seien die folgenden isomeren Formen der substituierten Hydroxylamine genannt:

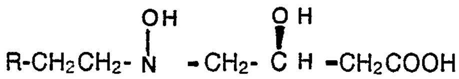

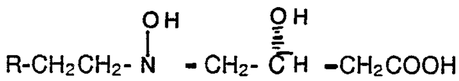

Insbesondere bevorzugt sind Verbindungen der Formel (I),
in welcher
R - für Wasserstoff, Methyl oder Ethyl steht, oder
- für ein Natrium- oder Kaliumion steht,
und
$R^1$ - für eine Gruppe der Formel

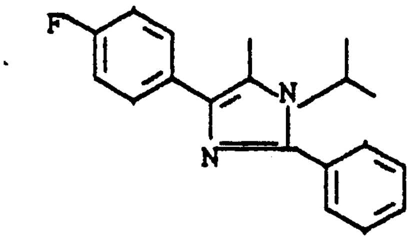

,

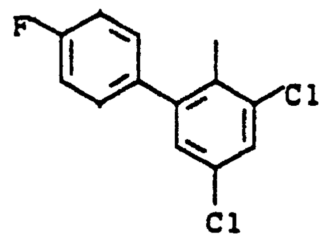

,

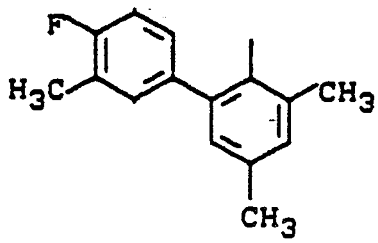

,

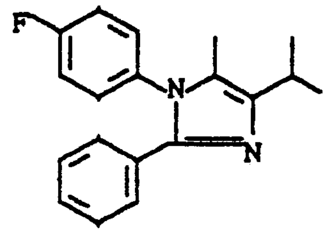

,

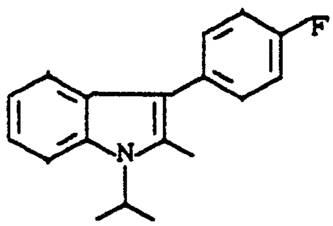

,

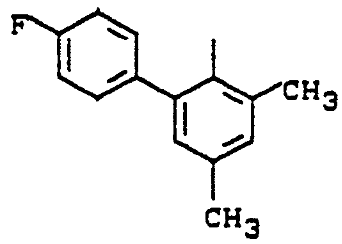

,

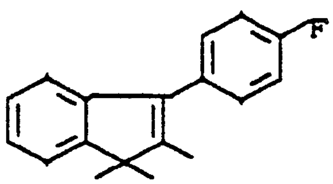

,

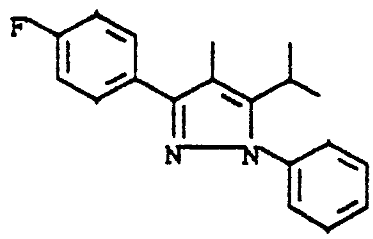

,

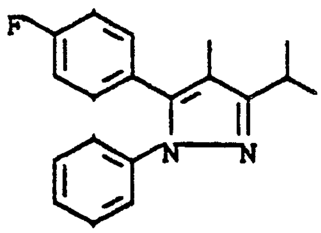

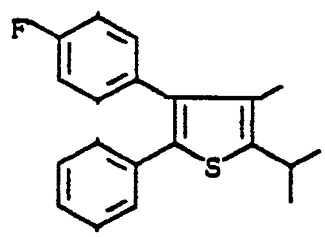

,

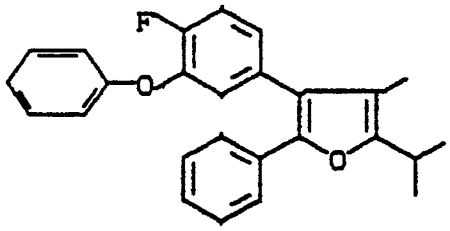 ,

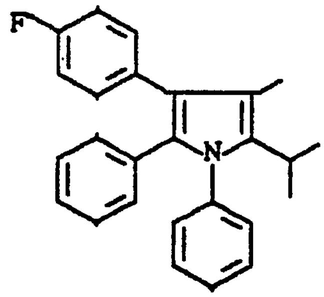 ,

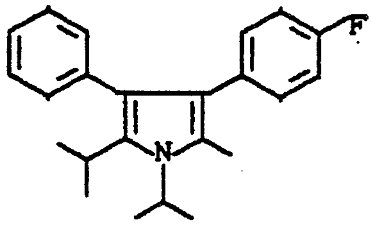 ,

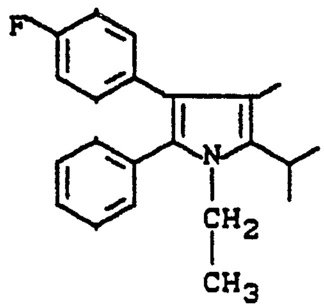 ,

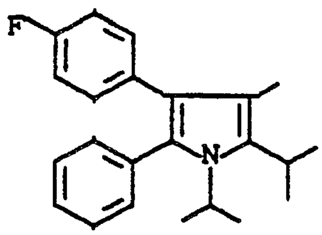 , steht,

Außerdem wurde ein Verfahren zur Herstellung der substituierten Hydroxylamine der allgemeinen Formel (I),

$$R^1\text{-}CH_2CH_2\text{-}\overset{OH}{\overset{|}{N}}\text{-}CH_2\overset{OH}{\overset{|}{C}}H\text{-}CH_2\text{-}COOR \qquad (I)$$

in welcher

R - für Wasserstoff,

- für einen physiologisch verträglichen Esterrest, oder
- für ein physiologisch verträglichen Kation steht,

und

$R^1$ - für eine Gruppe der Formel

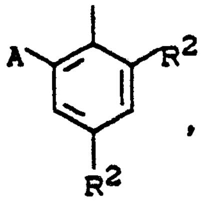 , 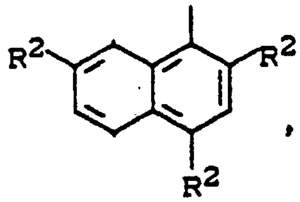 , 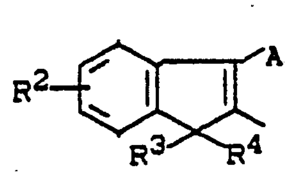 ,

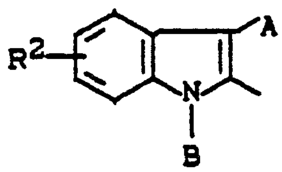 , 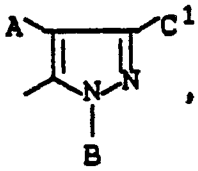 , 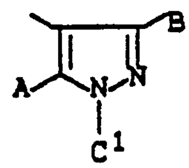 ,

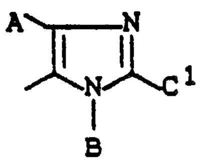 , 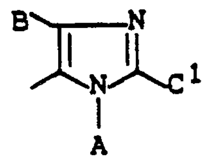 , 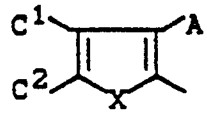 ,

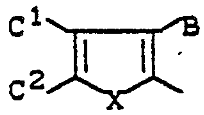 , 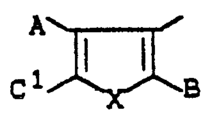 oder 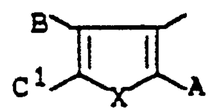

steht,

worin

$R^2$ - Wasserstoff, Halogen oder geradkettiges oder verzweigtes Alkyl mit bis zu 6 Kohlenstoffatomen bedeutet,

$R^3$, $R^4$ - gleich oder verschieden sind und

- Wasserstoff, Halogen oder geradkettiges oder verzweigtes Alkyl mit bis zu 6 Kohlenstoffatomen bedeuten,

oder

$R^3$ und $R^4$ gemeinsam eine Tetramethylenkette bilden,

A - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$,$R^6$-gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

B - Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ - gleich oder verschieden sind und- Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
X - O, S oder N-$C^3$ bedeutet,
und
$C^1$, $C^2$, $C^3$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -$NR^5R^6$,
worin
$R^5$ und $R^6$ die oben angegebene Bedeutung haben, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
oder
- Heteroaryl bedeutet, das bis zu 3-fach gleich oder verschieden durch Halogen, Alkyl, Alkoxy, Alkylthio, Alkylsulfonyl, Aryl, Aryloxy, Aryl thio, Arylsulfonyl, Trifluormethyl, Trifluormethoxy, Trifluormethylthio, Alkoxycarbonyl oder durch eine Gruppe der Formel -$NR^5R^6$ substituiert sein kann,
worin
$R^5$ und $R^6$ die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -$NR^5R^6$,
worin
$R^5$ und $R^6$ die oben angegebene Bedeutung haben,
gefunden,
das dadurch gekennzeichnet ist, daß man
Hydroxylamine der allgemeinen Formel (II)
$R^1$-$CH_2CH_2$-NH-OH (II)
in welcher
$R^1$ die oben angegebene Bedeutung hat,
mit Epoxiden der allgemeinen Formel (III)

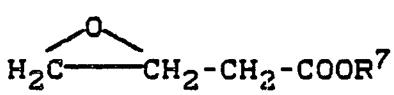 (III)

in welcher
$R^7$ - für geradkettiges oder verzweigtes Alkyl mit bis zu 6 Kohlenstoffatomen steht,
in inerten Lösemitteln umsetzt,
und dann im Fall der Herstellung der Säure, die Ester verseift,
und im Fall der Herstellung der Salze die Säuren mit den entsprechenden Basen umsetzt.

Das erfindungsgemäße Verfahren kann durch das folgende Formelschema erläutert werden:

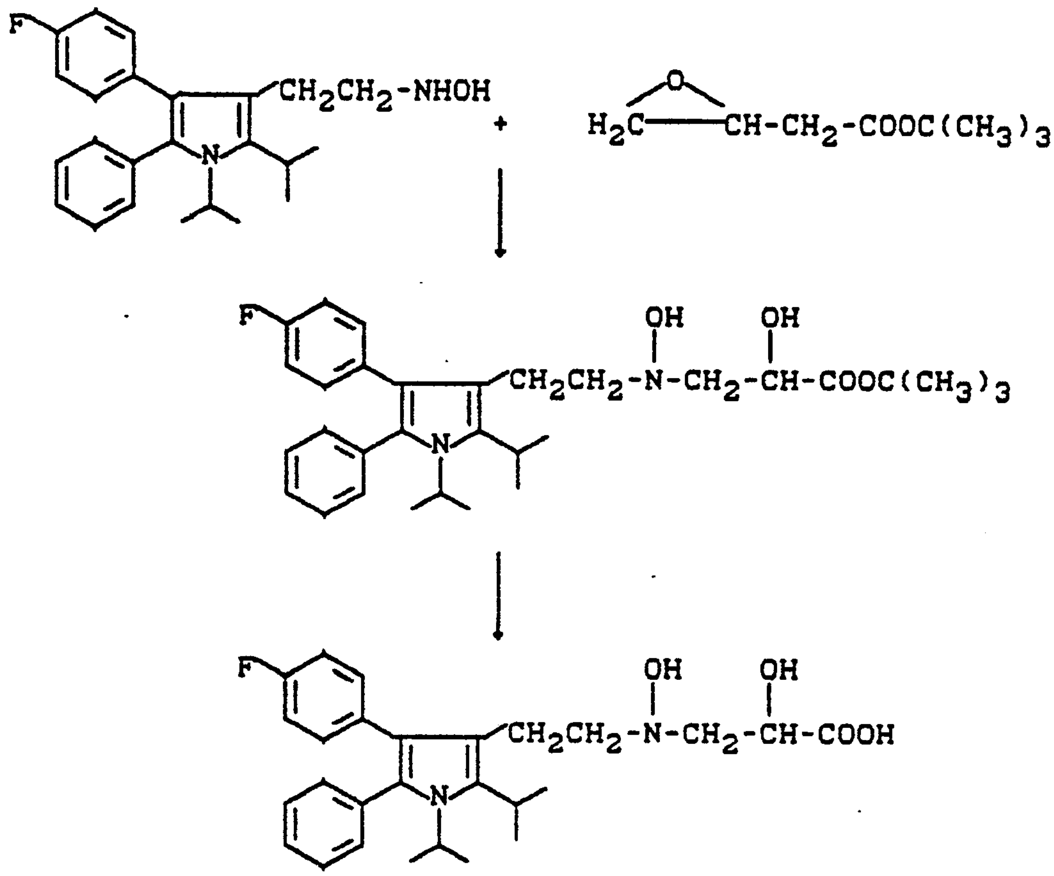

Als Lösemittel eignen sich hierbei die für eine Epoxidöffnung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Darüber hinaus ist es auch möglich, Hilfsstoffe wie Titan(IV)isopropylat oder Bortrifluorid-Etherat in aprotische Lösemittel wie beispielsweise Chlorkohlenwasserstoffe, bevorzugt Methylenchlorid oder aromatische Kohlenwasserstoffe wie Toluol, Xylol oder Benzol einzusetzen.

Die Reaktion wird im allgemeinen in einem Temperaturbereich von 0° C bis +150° C, bevorzugt von +20° C bis +100° C durchgeführt.

Im allgemeinen wird die Reaktion bei Normaldruck durchgeführt. Es ist aber auch möglich, bei Unterdruck oder bei Überdruck zu arbeiten (z.B. von 0,5 bis 5 bar).

Bei der Durchführung des erfindungsgemäßen Verfahrens wird das Epoxid im allgemeinen in einer Menge von 0,5 bis 2 Mol eingesetzt. Bevorzugt verwendet man molare Mengen der Reaktanden.

Zur Herstellung der erfindungsgemäßen Carbonsäuren (R = H) werden im allgemeinen die Carbonsäureester nach üblichen Methoden verseift. Die Verseifung erfolgt im allgemeinen indem man die Ester in inerten Lösemitteln mit üblichen Säuren behandelt, wobei im allgemeinen zunächst die Salze der erfindungsgemäßen Verbindungen entstehen, die anschließend in einem zweiten Schritt durch Behandeln mit Base in die freien Verbindungen überführt werden können.

Als Säuren können die üblichen Mineralsäuren, aber auch Carbonsäuren oder sulfonsäuren eingesetzt werden. Hierzu gehören bevorzugt anorganische Säuren wie beispielsweise Salzsäure oder Schwefelsäure, oder organische Carbonsäuren mit 1 bis 6 C-Atomen, gegebenenfalls substituiert durch Fluor, Chlor und/oder Brom, wie beispielsweise Essigsäure, Trifluoressigsäure, Trichloressigsäure oder Propionsäure, oder Sulfonsäure mit $C_1$-$C_4$-Alkylresten oder mit Arylresten wie beispielsweise Methansulfonsäure, Ethansulfonsäure, Benzolsulfonsäure oder Toluolsulfonsäure. Besonders bevorzugt wird die Verseifung mit Chlorwasserstoffsäure durchgeführt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders

bevorzugt werden Alkohole wie Methanol, Ethanol, Propanol oder Isopropanol verwendet. Ebenso ist es möglich, Gemische der genannten Lösemittel einzusetzen.

Besonders bevorzugt wird die Verseifung der tertiärbutylester in wässriger Salzsäure gegebenenfalls unter Zugabe eines Lösemittels wie beispielsweise Dioxan oder Tetrahydrofuran oder mit gasförmigen Chlorwasserstoff in Halogenkohlenwasserstoffen wie Methylenchlorid oder Chloroform durchgeführt.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Die als Ausgangsstoffe eingesetzten Epoxide der allgemeinen Formel (III) sind bekannt [J. Muggel, O.Vogl, J. Polym. Sci. Polym. Chem. Ed. 22, 2501-21 (1984); S. Boots, M.R. Boots, J. Pharm. Sci. 64, 1262 - 1264 (1975)] und können beispielsweise durch Umsetzung von But-3-en-carbonsäure-estern mit Persäuren hergestellt werden.

Als Epoxide seien beispielsweise genannt:

3,4-Epoxy-buttersäure-tert.-butylester
3,4-Epoxy-buttersäure-methylester
3,4-Epoxy-buttersäure-ethylester
3,4-Epoxy-buttersäure-propylester
3,4-Epoxy-buttersäure-isopropylester
3,4-Epoxy-buttersäure-isobutylester
3,4-Epoxy-buttersäure-n-butylester

Die als Ausgangsstoffe eingesetzten Hydroxylamine der Formel (II) sind neu.

Es wurde ein Verfahren zur Herstellung der Hydroxylamine der allgemeinen Formel (II)

$R^1$-$CH_2CH_2$-NH-OH (II)

in welcher

$R^1$ - für Wasserstoff,
- für einen physiologisch verträglichen Esterrest, oder
- für ein physiologisch verträgliches Kation steht,

gefunden, daß dadurch gekennzeichnet ist, daß man in einem ersten Schritt Aldehyde der allgemeinen Formel (IV)

R-CHO (IV)

in welcher

R die oben angegebene Bedeutung hat

mit Nitromethan in inerten Lösemitteln, gegebenenfalls in Anwesenheit von Hilfsmitteln umsetzt, und anschließend in einem zweiten Schritt die erhaltenen Nitroverbindungen der allgemeinen Formel (V)

R-CH=CH-$NO_2$ (V)

in inerten Lösemitteln gegebenenfalls in Anwesenheit eines Hilfsmittels reduziert.

Das erfindungsgemäße Verfahren kann durch folgendes Formelschema verdeutlicht werden:

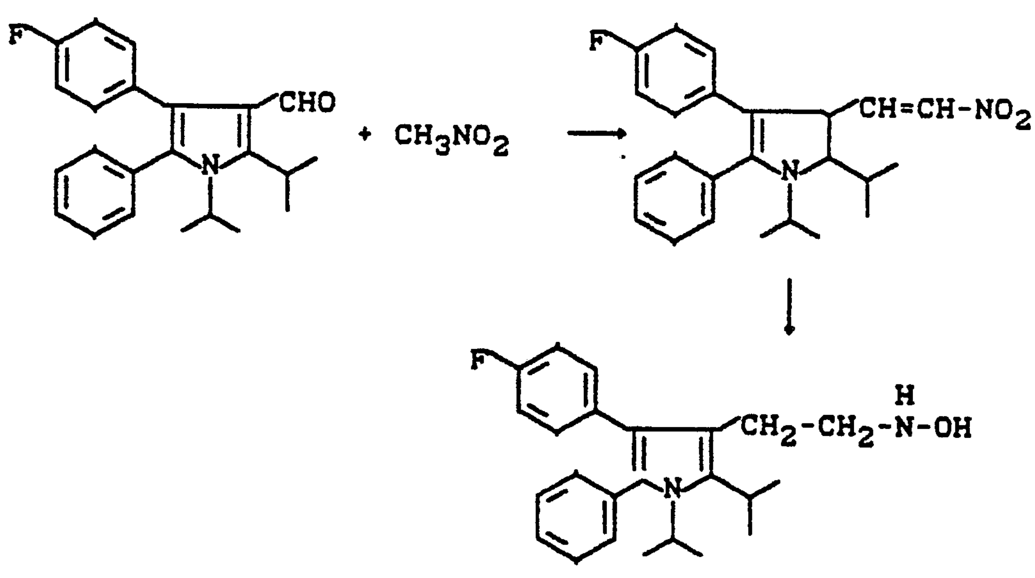

Als Lösemittel für den ersten Schritt eignen sich hierbei die üblichen organischen Lösemittel, die sich unter den Reaktionsbedingungen nicht verändern. Hierzu gehören bevorzugt Wasser oder Alkohole wie Methanol, Ethanol, Propanol oder Isopropanol, oder Ether wie Diethylether, Butylmethylether, Dioxan, Tetrahydrofuran oder Glykoldimethylether, oder Halogenkohlenwasserstoffe wie beispielsweise Methylenchlorid, Chloroform oder Tetrachlorkohlenstoff, oder Kohlenwasserstoffe wie Benzol, Toluol, Xylol, oder Erdölfraktionen, oder Amide wie Dimethylformamid oder Hexymethylphosphorsäuretriamid, oder Dimethylsulfoxid, Essigsäure, Trifluoressigsäure oder Nitroalkane. Ebenso ist es möglich, Gemische der genannten Lösemittel zu verwenden.

Als Hilfsmittel eignen sich Säuren oder Basen. Als Basen werden bevorzugt Alkali- oder Erdalkalihydroxide wie Na triumhydroxid, Kaliumhydroxid oder Bariumhyroxid, oder Alkalicarbonate wie Natriumcarbonat oder Kaliumcarbonat, oder Alkalialkoholate wie Natriummethanolat, Natriumethanolat, Kaliummethanolat, Kaliumethanolat, Kaliumtert.butanolat oder Ammoniak, oder Ammoniumacetat, oder organische Amine wie Diethylamin, Triethylamin, Diisopropylamin, Tripropylamin, Pyridin, Piperidin, Morpholin, N,N-Dimethylaminopyridin, DBN, DBU, Ethylendiamin oder N,N-Dimethylethylendiamin eingesetzt.

Als Säuren werden bevorzugt anorganische Säuren wie beispielsweise Salzsäure oder Schwefelsäure oder organische Carbonsäuren mit 1-6 Kohlenstoffatomen, gegebenenfalls substituiert durch Fluor oder Chlor, wie beispielsweise Ameisensäure, Essigsäure, Propionsäure, Chloressigsäure, Trichloressigsäure oder Trifluoressigsäure, oder Sulfonsäuren wie beispielsweise Methansulfonsäure, Ethansulfonsäure, Benzolsulfonsäure oder Toluolsulfonsäure eingesetzt.

Als besonders günstig hat es sich erwiesen der ersten Verfahrensschritt in Essigsäure als Lösemittel durchzuführen, gegebenenfalls in Anwesenheit von Piperidin oder Ammoniumacetat, oder in Nitromethan als Lösemittel und Ethylendiamin als Katalysator.

Der erste Schritt des Verfahrens wird im allgemeinen in einem Temperaturbereich von 0 °C bis +150 °C, bevorzugt von +20 °C bis +100 °C durchgeführt.

Die Umsetzung kann bei normalem, erhöhtem oder erniedrigtem Druck durchgeführt werden (z.B. 0,5 bis 5 bar). Im allgemeinen arbeitet man bei Normaldruck.

Bei der Durchführung des ersten Verfahrensschrittes wird im allgemeinen Nitromethan in einer Menge von 0,1 bis 100, bevorzugt von 0,5 bis 100, besonders bevorzugt von 1 bis 60 mol bezogen auf 1 mol des Aldehyds eingesetzt.

Der erfindungsgemäße Verfahrensschritt wird im allgemeinen durchgeführt, indem man den Aldehyd mit Nitromethan, gegebenenfalls in einem geeignetem Lösemittel und gegebenenfalls mit Basen mischt und gegebenenfalls erwärmt. Die Aufarbeitung erfolgt in üblicher Weise durch Extraktion, Chromatographie und/oder Kristallisation.

Die Reduktion im zweiten Reaktionsschritt wird im allgemeinen mit $BH_3$ als Reduktionsmittel, gegebenenfalls in Anwesenheit anderer Hydride als Katalysatoren in inerten Lösemitteln durchgeführt.

Als inerte Lösemittel eignen sich hierbei Ether wie Diethylether, Dioxan oder bevorzugt Tetrahydrofuran, oder Kohlenwasserstoffe wie Benzol, Toluol, Xylol, Hexan oder Cyclohexan.

Als Katalysatoren eignen sich i.a. Metallhydride wie beispielsweise Natriumborhydrid, Lithiumborhydrid oder Natriumcyanoborhydrid.

Besonders gut läßt sich die Reduktion mit $BH_3$ in Tetrahydrofuran in Anwesenheit einer katalytischen Menge Natriumborhyrid durchführen.

Die Reduktion erfolgt im allgemeinen in einem Temperaturbereich von -78 °C bis +40 °C, bevorzugt von -20 °C bis Raumtemperatur.

Das erfindungsgemäße Verfahren wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Im allgemeinen wird das Reduktionsmittel in einer Menge von 1 bis 5 mol, bevorzugt von 1 bis 2 mol bezogen auf 1 mol der Ketoverbindung eingesetzt.

Die als Ausgangsstoffe eingesetzten Aldehyde sind bekannt oder können nach bekannten Methoden hergestellt werden [G.E. Stokker et al., J. Med. Chem. 29, 170, (1986); EP 221 025; US-Patent 4 668 794; US-Patent 4 613 610; EP 114 027].

Als Aldehyde können beispielsweise erfindungsgemäß verwendet werden:

4′-Fluor-3,3′, 5-trimethyl-1,1′-5-phenyl-2-carboxaldehyd
4′-Fluoro-3,5-dimethyl-1,1′-biphenyl-2-carboxaldehyd
3,5-Dichloro-4′-fluor-1,1′biphenyl-2-carboxaldehyd
3-(4′-Fluorphenyl)-1-isopropylindol-2-carboxaldehyd
3-(4′-Fluorphenyl)-1H-indene-2-carboxaldehyd
3-(4′-Fluorphenyl)-3-isopropyl-1-phenyl-1H-pyrazol-4-carboxaldehyd

5-(4′-Fluorphenyl)-3-isopropyl-1-phenyl-1H-pyrazol-4-carboxaldehyd
4-(4′-Fluorphenyl)-1-isopropyl-2-phenyl-1H-imidazol-5- carboxaldehyd
1-(4′-Fluorphenyl)-4-isopropyl-2-phenyl-1H-imidazol-5-carboxaldehyd
3-(4-Fluorphenyl)-5-isopropyl-1-methyl-2-phenyl-pyrrol-4-carboxaldehyd
2-Formyl-1-(4-fluorphenyl)-3-isopropyl-5-phenyl-pyrrol
2-Formyl-3-(4-fluorphenyl)-1-isopropyl-5-phenyl-pyrrol
2-Formyl-3-(4-fluorphenyl)-1,5-diisopropyl-4-phenyl-pyrrol
3-Formyl-4-(4-fluorphenyl)-2-isopropyl-5-phenyl-furan
3-Formyl-4-(4-fluorphenyl)-2-isopropyl-5-phenyl-thiophen
2-tert.Butyl-3-formyl-4-(4-fluorphenyl)-5-phenyl-furan
3-Formyl-4-(4-fluorphenyl)-2,5-diphenyl-thiophen
3-Formyl-2-(4-fluorphenyl)-5-methyl-1-phenyl-4-isopropyl-pyrrol
3-Formyl-2-(4-fluorphenyl)-5-methyl-1-(2,6-dimethylphenyl)-4-isopropyl-pyrrol
3-Formyl-2-(4-fluorphenyl)-1-benzyl-4-isopropyl-5-methyl-pyrrol
4-(4-Chlorphenyl)-3-formyl-2-isopropyl-1-methyl-5-phenyl-pyrrol
3-Formyl-4-(4-fluor-3-phenoxy-phenyl)-2-isopropyl-1,5-dimethyl-pyrrol
3-Formyl-4-(4-fluor-3-phenoxy-phenyl)-2-isopropyl-5-phenyl-pyrrol
3-Formyl-4,5-bis-(4-fluorphenyl)-2-isopropyl-1-methyl-pyrrol
3-[3-(4-Fluorbenzyloxy)]-phenyl-3-formyl-2-isopropyl-1-methyl-4-phenyl-pyrrol
4-(4-Fluorphenyl)-3-formyl-2-isopropyl-5-phenyl-pyrrol
4-(4-Fluorphenyl)-3-formyl-1,2-diisopropyl-5-phenyl-pyrrol
4-(4-Fluorphenyl)-3-formyl-2-isopropyl-1,5-diphenyl-pyrrol
4-(4-Fluorphenyl)-3-formyl-2-isopropyl-1-(4-methoxyphenyl)-5-phenyl-pyrrol
1-(3-Benzyl-phenyl)-4-(4-fluorphenyl)-3-formyl-2-isopropyl-5-methyl-pyrrol

Die erfindungsgemäßen substituierten Hydroxylamine können in Arzneimitteln zur therapeutischen Behandlung beim Mensch und beim Tier verwendet werden. Bevorzugt können sie als Inhibitoren der 3-Hydroxy-3-methylglutaryl-Coenzym A (HGM-CoA) Reduktase und Inhibitoren der Cholesterolbiosynthese verwendet werden. Sie können deshalb zur Behandlung von Hyperlipoproteinämie, Lipoproteinämie oder Atherosklerose eingesetzt werden.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 98-Gew.-%, bevorzugt 1 bis 90 Gew.-%, der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.b: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral, parenteral, perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1

bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Herstellungsbeispiele

Beispiel 1

1-(4-Fluorphenyl)-4-methyl-pent-1-en-3-on

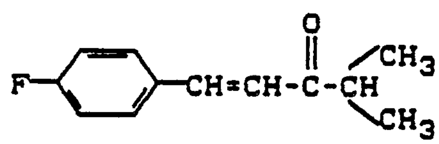

Zu 198,4 g (1,6 mol) frisch destilliertem 4-Fluorbenzaldehyd und 137,6 g (1,6 mol) Methylisopropylketon in 300 ml Methanol tropft man 75 ml 15%ige Kalilauge und rührt über Nacht bei Raumtemperatur. Dann wird mit 10 ml Essigsäure neutralisiert, 1 l Wasser zugesetzt und mit zwei 500 ml Portionen Ether extrahiert. Die vereinigten organischen Phasen werden mit 500 ml gesättigter Natriumchlorid-Lösung gewaschen und über Natriumsulfat getrocknet. Nach Abziehen des Lösemittels destilliert man im Hochvakuum.

Ausbeute: 198,6 g (65% der Theorie) gelbliches Öl

Kp.: 103 °C (0.3 mbar)

$^1$H-NMR ($CDCl_3$): $\delta$ = 1,2 (d, 6H, $CH_3$); 2,9 (sept, 1H, CH-($CH_3$)$_2$); 6,8 (d, 1H, Olefin-H); 7,1 (m, 2H, Aromaten-H); 7,6 (m, 3H, Aromaten-H + Olefin-H).

Beispiel 2

2-(4-Fluorphenyl)-5-methyl-1-phenyl-hexan-1,4-dion

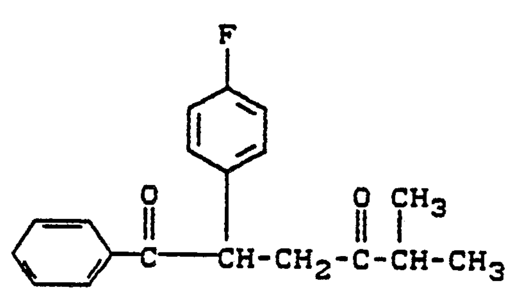

Zu 10,2 g (0,21 mol) Natriumcyanid in 500 ml Dimethylformamid, tropft man bei 35 °C eine Lösung von 110 g (1,04 mol) frisch destilliertem Benzaldehyd in 500 ml Dimethylformamid innerhalb von 30 min und rührt weitere 5 min bei dieser Temperatur. Dann werden innerhalb von 1,5 h 150 g (0,78 mol) 1-(4-Fluorphenyl)-4-methyl-pent-1-en-3-on (Beispiel 1) in 500 ml Dimethylformamid zugetropft und 1 h nachgerührt, wobei die Temperatur stets bei 35 °C gehalten wird.

Nach Versetzen mit 1,5 l Wasser wird viermal mit je 500 ml Chloroform extrahiert, die vereinigten organischen Phasen mit 800 ml 0,01 M $H_2SO_4$, 800 ml gesättigter Natriumhydrogencarbonat-Lösung und 1,5 l Wasser gewaschen und über Magnesiumsulfat getrocknet. Nach Abziehen des Lösemittels destilliert man im Hochvakuum.
Ausbeute: 167 g (72% der Theorie) farbloses Öl vom Sdp. : 165° C (0,1 mbar), das langsam erstarrt.
Schmp.: 54 - 55° C
$^1$H-NMR ($CDCl_3$): $\delta$ = 1,08 (d, 3H, $CH_3$); 1,12 (d, 3H, $CH_3$); 2,65 (sept, 1H, $CH-(CH_3)_2$); 2,7 (dd, 1H, $-CO-CH_2-CH$); 3,6 (dd, 1H, $-CO-CH_2-CH$); 5,12 (dd, 1H, $H-C-C_6H_4-F$); 6,95 (m, 2H, Aromaten-H); 7,23 (m, 2H, Aromaten-H); 7,4 (m, 3H, Aromaten-H); 7,95 (m, 2H, Aromaten-H).

## Beispiel 3

3-(4-Fluorphenyl)-1,5-diisopropyl-2-phenyl-pyrrol

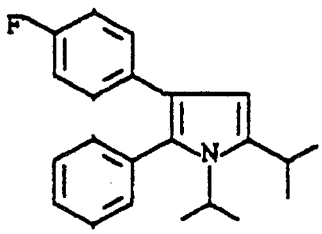

Zu 80 g (0,27 mol) Beispiel 2 und 96 g (1,63 mol) Isopropylamin in 300 ml Toluol tropft man bei 10° C eine Lösung von 56 g (0,29 mol) Titantetrachlorid in 200 ml Toluol und rührt 5 h bei RT. Man filtriert über Kieselgur, wäscht mit heißem Toluol nach und extrahiert die organischen Phasen mit Wasser, 1 N-Salzsäure, gesättigter Natriumhydrogencarbonat-Lösung. Nach Abziehen des Toluols bleibt ein Rückstand, der aus Petrolether umkristallisiert wird.
Ausbeute: 51 g farblose Kristalle
Schmp.: 147° C

## Beispiel 4

3-(4-Fluorphenyl)-4-formyl-1,5-diisopropyl-2-phenyl-pyrrol

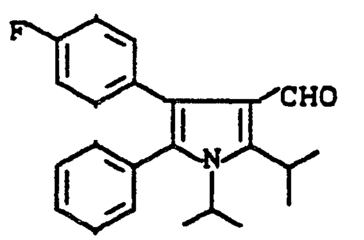

Zu einer Suspension von 38,6 g (0,12 mol) der Verbindung aus Beispiel 3 in 140 g (1,92 mol) Dimethylformamid tropft man vorsichtig 11 g (0,12 mol) Phosphoroxychlorid, rührt 1 h bei Raumtemperatur, dann 30 min bei 60° C, bis sich alles gelöst hat.
Man gießt auf 1 l kalte 0,1 M Natronlauge, gibt noch 150 ml 1 M Natronlauge hinzu und rührt 15 min bei Raumtemperatur (pH 7). Es wird 4 x mit je 250 ml Dichlormethan extrahiert, die organischen Phasen mit je 300 ml gesättigter Natriumhydrogencarbonat-Lösung und Wasser gewaschen und über Natriumsulfat getrocknet.

Man engt zu einem Kristallbrei ein, der aus Ethanol in mehreren Kristallisationen 35 g (85 %) gelbliche Kristalle ergibt.
F = 197° C

Beispiel 5

1-[3-(4-Fluorphenyl)-1,5-diisopropyl-2-phenyl-pyrrol-4-yl]-2-nitro-ethen

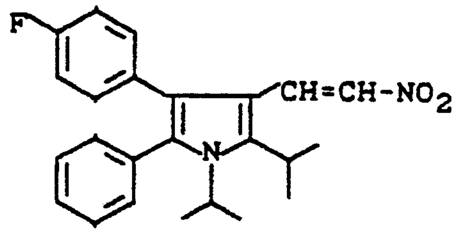

18,1 g (51,8 mmol) der Verbindung aus Beispiel 4 werden in 140 g Nitromethan mit 0,53 g (8,83 mmol) 1,2-Diaminoethan 1,5 h bei 75° C gerührt.
Nach dem Abkühlen wird abgesaugt und mit Petrolether gewaschen.
Ausbeute: 19,8 g (97 % d.Th.) gelbe Kristalle
F° C 256° C (aus Dichlormethan, Petrolether)
$^1$H-NMR ($CDCl_3$): δ = 1,45 (d, 6H, C-isopropyl-$CH_3$); 1,6 (d, 6H, N-isopropyl-$CH_3$); 3,6 (sept, 1H, C-isopropyl-H); 4,9 (sept, 1H, N-isopropyl-H); 6,5 (d, 1H, Olefin-H); 6,8 - 7,3 (m, 9H, Aromaten-H); 8,4 (d, 1H, Olefin-H).

Beispiel 6

N-{2-[3-(4-Fluorphenyl)-1,5-diisopropyl-2-phenyl-pyrrol-4-yl]-ethyl}-hydroxylamin

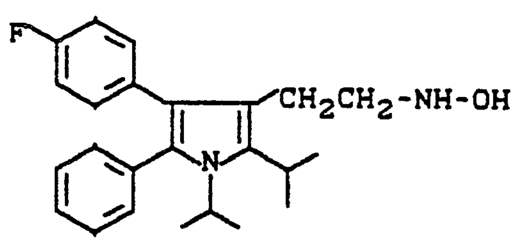

Zu 19,1 ml (0,19 mol) einer 1 M Lösung von Borwasserstoffsäure in Tetrahydrofuran tropft man bei 0° C unter Argon eine Lösung von 30 g (76,5 mmol) der Verbindung aus Beispiel 5 in 200 ml warmem Tetrahydrofuran, gibt dann 0,5 g (13,2 mmol) Natriumborhydrid zu und rührt 2 h bei Raumtemperatur. Dann werden vorsichtig 180 ml kaltes Wasser zugetropft und 1 h unter Rückfluß gekocht. Es wird mit 10 ml 6 N Natronlauge auf pH = 13 eingestellt, dreimal mit je 200 ml Essigester extrahiert, die vereinigten organischen Phasen über Natriumsulfat getrocknet und zur Trockene eingeengt. Der Rückstand wird an 500 g Kieselgel (230-400 mesh) mit Dichlormethan, Dichlormethan-Methanol (10:1) und Dichlormethan-Methanolkonz. Ammoniak (100:10:1) chromatographiert, bis das Hauptprodukt mit niedrigstem $R_f$-Wert austritt.
Ausbeute: 13,5 g (46 % d.Th.), farblose Kristalle vom Schmelzpunkt 182° C (aus Dichlormethan/Methanol).
$^1$H-NMR ($CDCl_3$): δ = 1,4 (m, 12H, isopropyl-$CH_3$); 2,7 (m, 4H, $CH_2$); 3,4 (sept, 1H, C-isopropyl-H); 4,45 (m, 1H, N-isopropyl-H); 4,9 (b, 2H); 6,8 - 7,3 (m, 9H, Aromaten-H).

Beispiel 7

N-{2-[3-(4-Fluorphenyl)-1,5-diisopropyl-2-phenyl-pyrrol-4-yl]-ethyl}-N-(3-tertiärbutoxycarbonyl-2-hydroxypropyl)-hydroxylamin

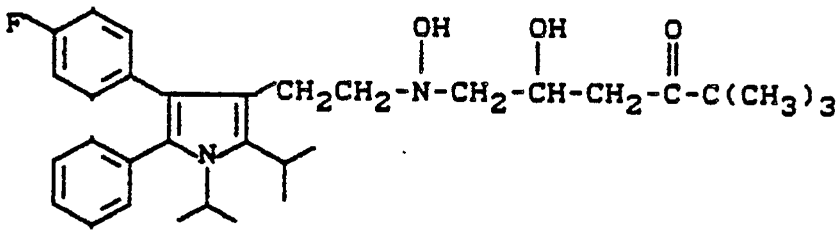

0,9 g (2,37 mol) Beispiel 6 werden zusammen mit 0,37 g (2,37 mmol)3,4-Epoxy-buttersäure-tertiärbutylester in 30 ml absolutem Ethanol 24 h am Rückfluß gekocht. Nach Abziehen des Lösemittels wird an 50 g Kieselgel 230-400 mesh in einer Säule von 1,5 cm Durchmesser mit Petrolether/Essigester 5:1 chromatographiert. Man erhält 0,9 g farblosen Schaum.
$^1$H-NMR ($CDCl_3$): $\delta$ = 1,45 (s, 9H, t-butyl); 2,3 (d, 2H, $CH_2$-COOR); 2,45 - 2,65 (m, 4H, $CH_2$); 2,9 (m, 2H, $CH_2$); 3,4 (m, b, 2H, C-isopropyl-H und OH); 4,15 (m, 1H, H-C-OH); 4,45 (sept, 1H, N-isopropyl-H); 4,95 (b, 1H, OH); 6,8 - 7,25 (m, 9H, Aromaten-H).

Beispiel 8

N-{2-[3-(4-Fluorphenyl)-1,5-diisopropyl-2-phenyl-pyrrol-4-yl]-ethyl}-N-(3-carboxy-2-hydroxy-propyl)-hydroxylamin-hydrochlorid

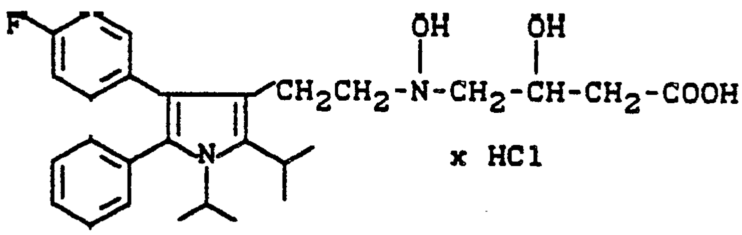

1,25 g (2,32 mmol) Beispiel 7 werden in 70 ml 1N Salzsäure 3 h am Rückfluß gekocht. Nach dem Einengen wird über Phosphorpentoxid getrocknet.
Ausbeute: 1,17 g (98%) leicht gefärbter Schaum.
$R_f$: 0,4 (Chloroform-Methanol (2:1) auf Kieselgel)
$^1$H-NMR ($CDCl_3$): $\delta$ = 1,4 (m, 12H, isopropyl-$CH_3$); 2,35 (m, 2H, $CH_2$); 2,8 - 3,2 (m, 6H, $CH_2$); 3,4 (m, 1H, C-isopropyl-H); 4,45 (m, 1H, N-isopropyl-H); 3,9 -5,2 (b, OH, NH, (HO)-CH).

Beispiel 9

1-Ethyl-3-(4-fluorphenyl)-5-isopropyl-2-phenyl-pyrrol

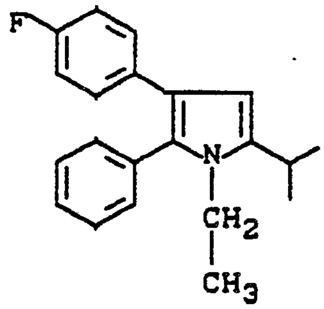

Zu einer siedenden Mischung von 8,2 g (27,5 mmol) Beispiel 2 in 25 ml 50%igem wäßrigem Ethylamin gibt man soviel Ethanol, daß eine klare Lösung entsteht und kocht 1 h am Rückfluß. Das Ethanol wird abgezogen, der Rückstand zwischen Wasser und Chloroform verteilt, die organische Phase mit gesättigter Natriumchloridlösung gewaschen und über Natriumsulfat getrocknet. Nach dem Einengen zur Trockene erhält man 4,5 g (53%) farblose Kristalle vom Schmp.: 110 °C.

$^1$H-NMR ($CDCl_3$): δ = 1,1 (t, 3H, $CH_3$); 1,35 (d, 6H, isopropyl-$CH_3$); 3,0 (sept, 1H, isopropy-H); 3,85 (q, 2H, $CH_2$); 6,15 (s, 1H, 4-H); 6,7 - 7,4 (m, 9H, Aromaten-H).

Beispiel 10

N-{2-[1-Ethyl-3-(4-fluorphenyl)-5-isopropyl-2-phenyl-pyrrol-4-yl]-ethyl}-N-(3-tertiärbutoxycarbonyl-2-hydroxypropyl)-hydroxylamin

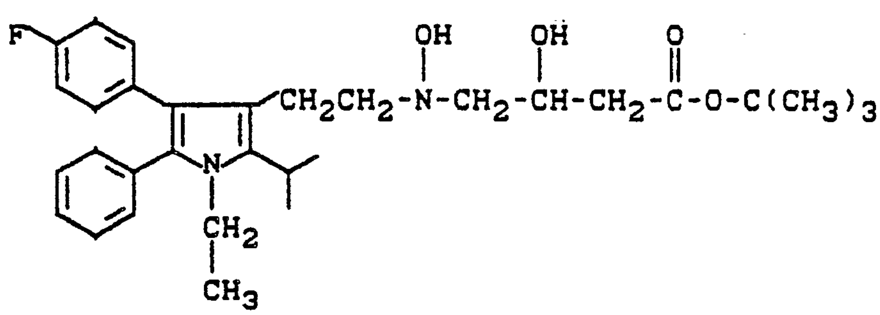

Die Verbindung wird in Analogie zu den Beispielen 4 -7 aus der Verbindung aus Beispiel 9 erhalten. Farbloser Schaum.

$^1$H-NMR ($CDCl_3$): δ = 1,1 (t, 3H, $CH_2$-$CH_3$); 1,45 (m, 15H, isopropyl-$CH_3$ und tertiärbutyl-$CH_3$); 2,35 (d, 2H, $CH_2$-COOR); 2,55 - 2,9 (m, 6H, $CH_2$); 3,2 (m, 1H, isopropyl-H); 3,9 (q, 2H, $CH_2$-$CH_3$); 4,15 (m, 1H, (HO)-CH); 6,8 - 7,3 (m, 9H, Aromaten-H).

Beispiel 11

N-{2-[1-Ethyl-3-(4-fluorphenyl)-5-isopropyl-2-phenyl-pyrrol-4-yl]-ethyl}-N-(3-carboxy-2-hydroxypropyl)-hydroxylamin-hydrochlorid

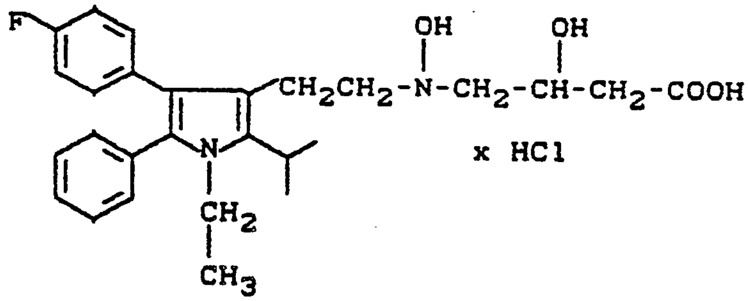

Die Verbindung wird analog Beispiel 8 aus der Verbindung aus Beispiel 10 erhalten.
Farbloser Schaum
$R_f$ = 0,2 (Chloroform/Methanol 5:1)

Beispiel 12

3-(4-Fluorphenyl)-5-isopropyl-1,2-diphenyl-pyrrol

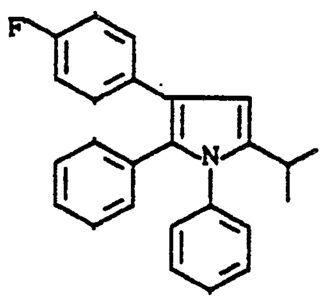

10,1 g (34 mmol) 2-(4-Fluophenyl)-5-methyl-1-phenyl-hexan-1,4-dion (Beispiel 2) und 9,3 g (102 mmol) Anilin werden in 150 ml Toluol unter Zugabe von 500 mg p-Toluolsulfonsäure 24 h am Wasserabscheider unter Rückfluß erhitzt. Nach Abkühlen und Verdünnen mit Essigsäureethylester wird mit 1 N-Salzsäure und anschließend mit Natriumhydrogencarbonat-Lösung gewaschen, über Magnesiumsulfat getrocknet und im Vakuum eingeengt. Abschließend wird über Keiselgel chromatographiert.
Ausbeute: 4,4 g (37% der Theorie)
$^1$H-NMR ($CDCl_3$): $\delta$ = 1,17 (d, 6H); 2,70 (sept., 1H); 6,28 (s, 1H); 6,80 - 7,30 (m, 14H).

Beispiel 13

N-{2-[3-(4-Fluorphenyl)-5-isopropyl-1,2-diphenyl-pyrrol-4-yl)-ethyl}-N-(3-tertiärbutoxycarbonyl-2-hydroxypropyl)-hydroxylamin

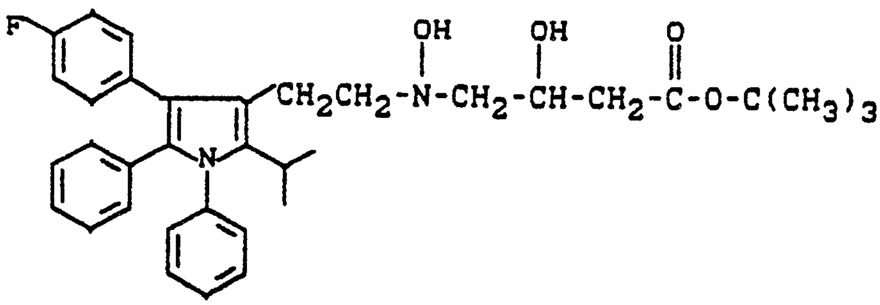

Die Verbindung wird analog Beispiel 4 - 7 aus der Verbindung aus Beispiel 12 enhalten.
Farbloser Schaum.
$^1$H-NMR ($CDCl_3$): $\delta$ = 1,25 (d, 6H, isopropyl-$CH_3$); 1,45 (s, 9H, t-but.-$CH_3$); 2,4 (d, 2H, $CH_2$-COOR); 2,5 - 2,75 (m, 4H, $CH_2$); 2,95 (m, 2H, $CH_2$ und isopropyl-H); 3,3 (b, 1H, OH); 4,15 (m, 1H, (OH)-CH); 5,1 (b, 1H, OH); 6,7 - 7,35 (m, 14H, Aromaten-H).

## Beispiel 14

N-{2-[1-Ethyl-3-(4-fluorphenyl)-5-isopropyl-2-phenyl-pyrrol-4-yl]-ethyl}-N-(3-carboxy-2-hydroxypropyl)-hydroxylamin-hydrochlorid

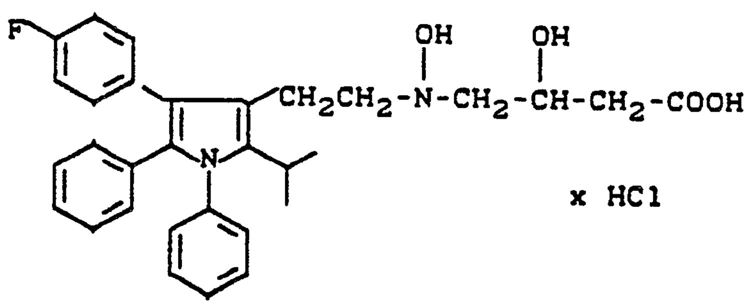

Die Verbindung wird analog Beispiel 8 aus der Verbindung aus Beispiel 13 mit 1 N Salzsäure, die 20% Dioxan enthält, erhalten.
Farbloser Schaum
$R_f$ = 0,3 (Chloroform / Methanol 3:1)

## Beispiel 15

N-[2-(2,4-Dimethylphenyl)-ethyl]-N-(3-carboxy-2-hydroxypropyl)-hydroxylamin-hydrochlorid

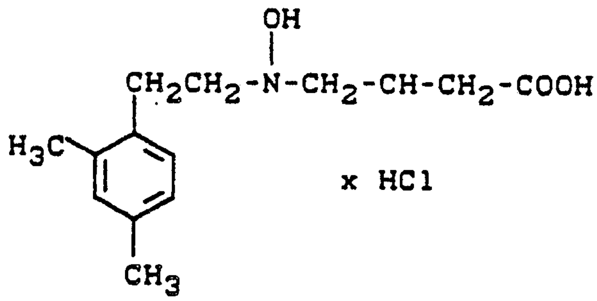

Die Verbindung erhält man analog Beispiel 5 - 8 aus 2,4-Dimethyl-benzaldehyd.
Farbloser Schaum.
$^1$H-NMR ($d_6$-DMSO + $D_2O$); δ = 2,2 (m, 6H, $CH_3$); 2,45 (m, überlagert durch DMSO-Signal, $CH_2$); 2,95 (m, 2H, $CH_2$); 3,35 (m, 4H, $CH_2$); 4,3 (m, 1H, HO-<u>CH</u>); 7,0 (m, 3H, Aromaten-H).

Beispiel 16

N-[2-(2,4-Dimethylphenyl)-ethyl]-N-(3-methoxycarbonyl-2-hydroxy-propyl)-hydroxylamin

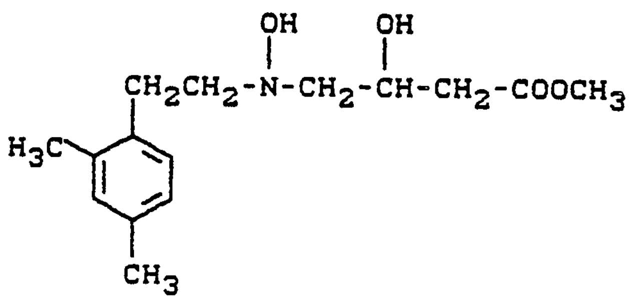

50 mg (0,19 mmol) der Verbindung aus Beispiel 15 kocht man 2 h in 5 ml Methanol, das 3 Tropfen konzentrierte Schwefelsäure enthält, am Rückfluß. Es wird zur Trockene eingeengt, der Rückstand zwischen Essigester und gesättigter Natriumhydrogencarbonat-Lösung verteilt, die organische Phase über Natriumsulfat getrocknet und das Lösemittel im Vakuum abgezogen.
Ausbeute: 40 mg (75%) farbloses Öl.
MS: m/e = 281 (15%, $M^+$)); 233 (20%, M-$OCH_3$-OH); 162 (100%, M-$C_9H_{11}$).

Beispiel 18

N-[2-(2,4-Dichlorphenyl)-ethyl]-N-(3-tertiärbutoxycarbonyl-2-hydroxy-propyl)-hydroxylamin

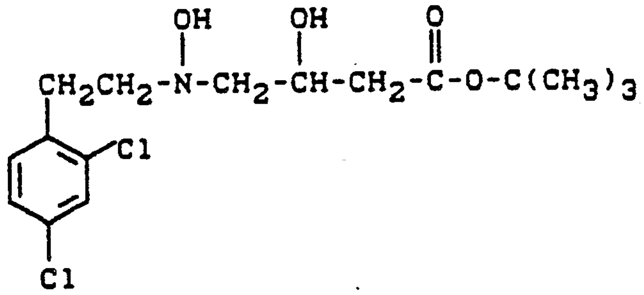

Die Verbindung erhält man analog Beispiel 5 - 7 aus 2,4-Dichlorbenzaldehyd.
Farbloses Öl.
$^1H$-NMR ($CDCl_3$): $\delta$ = 1,45 (s, 9H, $C(CH_3)_3$); 2,4 (d, 2H, $CH_2$); 2,5 (d, 2H, $CH_2$); 3,0 (m, 4H, $CH_2$); 4,3 (m, 1H, HO-CH); 5,9 (b, 1H, OH); 7,15 - 7,3 (m, 3H, Aromaten-H).

Beispiel 18

N-[2-(2,4-Dichlorphenyl)-ethyl]-N-(3-carboxy-2-hydroxy-propyl)-hydroxylamin-hydrochlorid

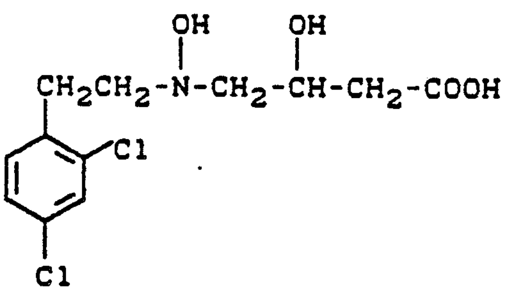

Man erhält die Verbindung analog Beispiel 8 aus der Verbindung aus Beispiel 17.
Farbloser Schaum.

| Elementaranalyse: | | | | | | | | |
|---|---|---|---|---|---|---|---|---|
| Ber.: | C | 41,8 | H | 4,7 | Cl | 30,9 | N | 4,1 |
| Gef.: | C | 41,4 | H | 4,8 | Cl | 30,0 | N | 4,0 |

Beispiel 19

N-[2-(4′-Fluor-3,5-dimethyl-1,1′-biphen-2-yl)-ethyl]-N-(3-tertiärbutoxycarbonyl-2-hydroxy-propyl)-hydroxylamin

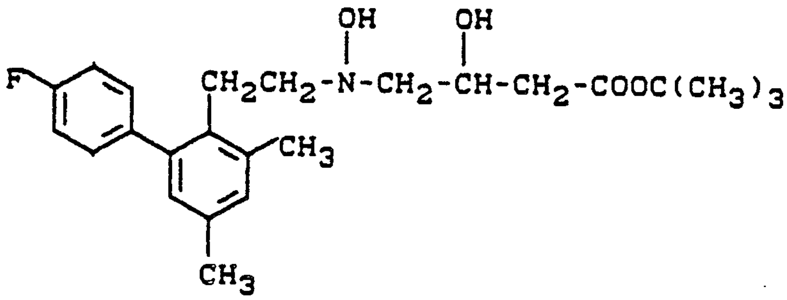

Die Verbindung erhält man analog Beispiel 5 - 7 aus 4′-Fluor-3,5-dimethyl-1,1′-biphenyl-2-carboxaldehyd (G.E. Stokker et al., J. Med. Chem. 29, 170 (1986)).
$^1$H-NMR ($CDCl_3$): δ = 1,45 (s, 9H, $C(CH_3)_3$); 2,25 - 2,4 (m, 8H, $CH_3$ und $CH_2$); 2,55 (m, 2H, $CH_2$); 2,65 (m, 2H, $CH_2$); 2,8 (m, 2H, $CH_2$); 3,6 (b, 1H, OH); 4,15 (m, 1H, HO-CH); 4,9 (b, 1H, OH); 6,8 - 7,3 (m, 6H, Aromaten-H).

Beispiel 20

N-[2-(4′-Fluor-3,5-dimethyl-1,1′-biphen-2-yl)-ethyl]-N-(3-carboxy-2-hydroxy-propyl)-hydroxylamin-hydrochlorid

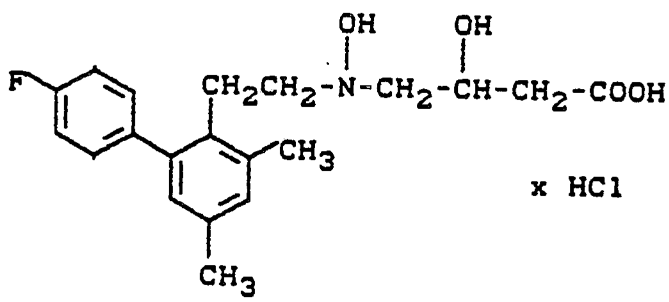

x HCl

Die Verbindung erhält man analog Beispiel 8 aus Beispiel 19.
Farbloses Öl.
$R_f$ = 0,38 (Chloroform / Methanol 4:1)

Beispiel 21

2-(4-Fluor-3-Phenoxy-benzyliden)-4-methyl-3-oxo-pentan-carbonsäure-ethylester

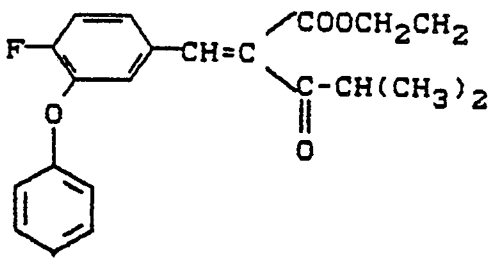

Zu einer Lösung von 79 g (0,5 mol) 4-Methyl-3-oxo-pentan-carbonsäure-ethylester (hergestellt analog dem Verfahren von S.B. Soloway und F.B. La Forge, J. Am. Chem. Soc. 69, 2677 (1947) aus Methylisopropylketon) und 108g (0,5 mol) 4-Fluor-3-phenoxy-benzaldehyd in 300 ml Isopropanol gibt man 2,8 ml (29 mmol) Piperidin und 1,7 ml (29 mmol) Essigsäure und rührt über Nacht bei RT. Dann wird eingeengt, der Rückstand in 500 ml Ether aufgenommen, zweimal mit Wasser und einmal mit gesättigter NaCl-Lösung gewaschen, über Natriumsulfat getrocknet und das Lösemittel abgezogen. Den Rückstand destilliert man im Hochvakuum bis bei 185 °C und 0,3 mbar die letzte Fraktion übergeht. Alle Destillate (44 g) werden verworfen. Der Destillationsrückstand enthält das Produkt: bräunliches Öl (144,7 g, 81%), das ohne Reinigung weiter umgesetzt wird.
$R_f$ = 0,25 (Petrolether/Essigester 10:1).

Beispiel 22

3-Ethoxy-carbonyl-2-(4-fluor-3-phenoxy-phenyl)-5-methyl-1-phenyl-hexan-1,4-dion

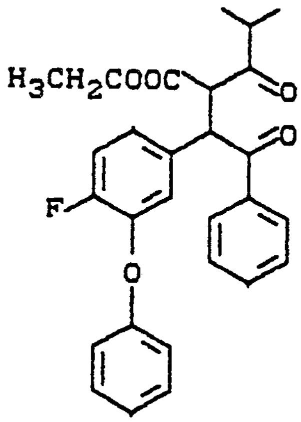

142,4 g (0,4 mol) Beispiel 21 werden zusammen mit 42,4 g (0,4 mol) Benzaldehyd, 10,8 g (0,04 mol) 3-Benzyl-5-(2-hydroxyethyl)-4-methyl-thiazoliumchlorid und 33 ml Triethylamin in 270 ml Ethanol über Nacht am Rückfluß gekocht. Das Lösemittel wird im Vakuum abgezogen, der Rückstand in Chloroform gelöst, zweimal mit 1 N Schwefelsäure, mit Wasser und gesättigter Bicarbonat-Lösung gewaschen und über Natriumsulfat getrocknet. Nach dem Einengen zur Trockene bleiben 185,2 g gelbliches Öl. Das Rohprodukt ist genügend rein zur Weiterverarbeitung.
$R_f$ = 0,17 (Petrolether/Essigester 10:1)

Beispiel 23

3-Ethoxycarbonyl-4-(4-fluor-3-phenoxy-phenyl)-2-isopropyl-5-phenyl-furan

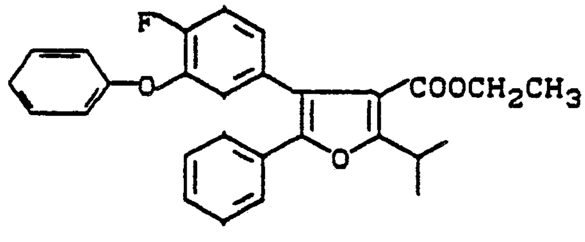

185 g (0,4 mmol) der Verbindung aus Beispiel 22 werden mit 15,2 g (0,05 mol) p-Toluolsulfonsäure-hydrat in 2 l Toluol 2,5 h am Wasserabscheider gekocht. Es scheiden sich ca. 7 ml Wasser ab. Nach dem Abkühlen wäscht man zweimal mit gesättigter Bicarbonat-Lösung und einmal mit Kochsalzlösung, trocknet über Natriumsulfat und engt zur Trockene ein. Der Rückstand wird aus Ethanol umkristallisiert.
Ausbeute: 57K9 g (33%) farblose Kristalle.
Schmp.: 99 °C
$^1$H-NMR ($CDCl_3$): $\delta$ = 1,1 (t, 3H, O-$CH_2$-$CH_3$); 1,35 (d, 6H, isopropyl-$CH_3$); 3,8 (sept, 1H, isopropyl-H); 4,15 (q, 2H, O-$CH_2$-$CH_3$); 6,1 - 7,35 (m, 13H, Aromaten-H).

Beispiel 24

4-(4-Fluor-3-phenoxy-phenyl)-3-hydroxymethyl-2-isopropyl-5-phenyl-furan

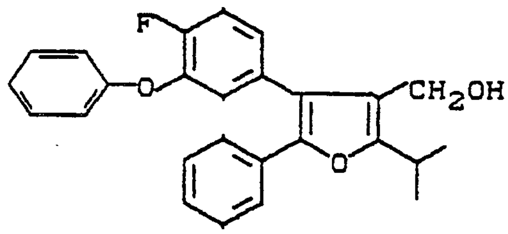

Zu einer Lösung von 31 g (70 mmol) der Verbindung aus Beispiel 23 in 350 ml wasserfreiem Toluol tropft man bei -78 °C unter Argonatomosphäre 70 ml (84 mmol) einer 1,2 M Diisobutylaluminiumhydrid-Lösung in Toluol langsam zu, so daß die Temperatur -65 °C nicht übersteigt. Nach 1 h Rühren bei -70 °C gibt man weitere 58 ml (70 mmol) DIBAH-Lösung zu und rührt noch 1 h. Dann werden bei ca. -30 °C 240 ml 1 N Salzsäure zugetropft und schließlich 400 ml Wasser und 200 ml Essigester zugegeben, wobei sich die Mischung auf Raumtemperatur erwärmt. Die wäßrige Phase wird noch 3 mal mit 250 ml Essigester extrahiert, die gewaschenen organischen Phasen mit 400 ml Wasser und 400 ml gesättigter Natriumchlorid-Lösung gewaschen und über Natriumsulfat getrocknet. Man zieht das Lösemittel ab und trocknet im Hochvakuum.
Ausbeute: 27,2 g (97%)
Farblose Kristalle
Schmp.: 157 °C

Beispiel 25

4-(4-Fluor-3-phenoxy-phenyl)-3-formyl-2-isopropyl-5-phenyl-furan

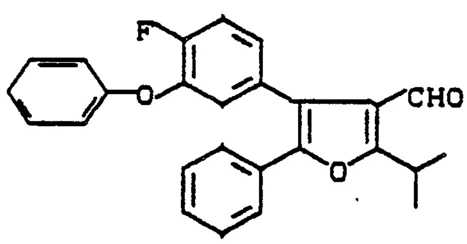

Zu 9,2 ml (130 mmol) Dimethylsulfoxid in70 ml wasserfreiem Dichlormethan tropft man bei -70 °C bis -65 °C eine Lösung von 13,8 ml (97 mmol) Trifluoressigsäureanhydrid in 90 ml Dichlormethan und rührt 10 Minuten bei dieser Temperatur. Dann werden 26,1 g (665 mmol) der Verbindung aus Beispiel 24, gelöst in

500 ml Dichlormethan zugetropft und noch 1 h bei -65 °C gerührt. Nun gibt man 26,9 ml (195 mmol) Triethylamin zu und hält weitere 10 Minuten bei derselben Temperatur. Nach dem Erwärmen auf RT wäscht man mit gesättigter Bicarbonat- und Kochsalzlö sung, trocknet über Natriumsulfat und zieht das Lösemittel im Vakuum ab. Nach dem Auskochen des Rückstandes mit Ethanol bleiben 17,6 g (68%) farbloser Feststoff vom Schmp.: 137 °C. Beim Abkühlen der ethanolischen Lösung fallen weitere 5,6 g (21%) vom Schmp.: 138 °C an.

$^1$H-NMR ($CDCl_3$): $\delta$ = 1,4 (d, 6H, Isopropyl-$CH_3$); 3,7 (sept, 1H, isopropyl-H); 6,9 - 7,4 (m, 14H, Aromaten-H); 9,8 (s, 1H, CHO).

Beispiel 26

N-{2-[4-(4-Fluor-3-phenoxy-phenyl)-2-isopropyl-5-phenyl-furan-3-yl]-ethyl}-N-(3-tertiärbutoxycarbonyl-2-hydroxy-propyl)-hydroxylamin

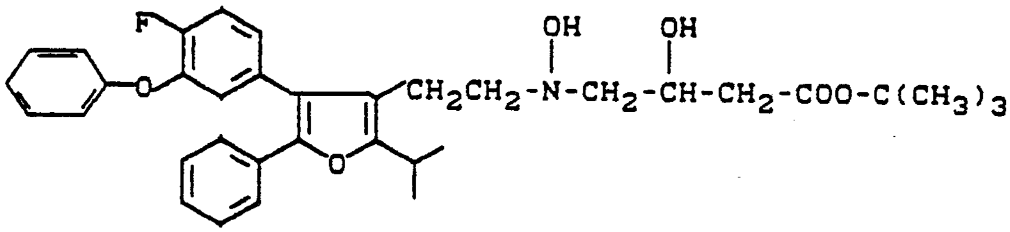

Die Verbindung erhält man analog Beispiel 5 - 7 aus der Verbindung aus Beispiel 25.

Farbloser Schaum.

$^1$H-NMR ($CDCl_3$): $\delta$ = 1,3 (d, 6H, isopropyl-$CH_3$); 1,45 (s, 9H, tert.butyl-$CH_3$); 2,4 (d, 2H, $CH_2$); 2,6 (m, 6H, $CH_2$); 3,1 (m, 1H, isopropyl-H); 3,4 (b, 1H, OH); 4,2 (1H, HO-CH); 5,2 (b, 1H, OH); 6,9 - 7,4 (m, 14H, Aromaten-H).

Beispiel 27

N-{2-[4-(4-Fluor-3-phenoxy-phenyl)-2-isopropyl-5-phenyl-furan-3-yl]-ethyl}-N-(3-carboxy-2-hydroxy-propyl)-hydroxylamin-hydrochlorid

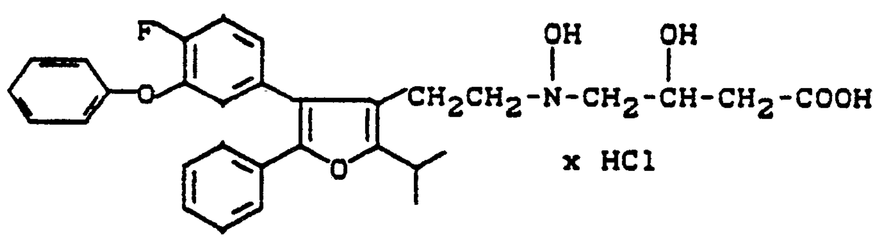

Die Verbindung erhält man analog Beispiel 8 aus der Verbindung aus Beispiel 26.

Fester farbloser Schaum.

$^1$H-NMR ($D_6$-DMSO): $\delta$ = 1,3 (d, 6H, isopropyl-$CH_3$); 2,4 (m, überlagert von DMSO-Signal, $CH_2$); 2,8 (m, 2H, $CH_2$); 3,0 - 3,3 (b, überlagert von $H_2O$-Signal, $CH_2$); 4,25 (b, 1H, HOCH); 5,4 (sehr b, 1H, OH); 6,9 - 7,6 (m, 14H, Aromaten-H).

Beispiel 28

N-{2-[4-(4-Fluorphenyl)-2-isopropyl-5-phenyl-thiophen-3-yl]-ethyl}-N-(3-tert.butoxycarbonyl-2-hydroxy-propyl)-hydroxylamin

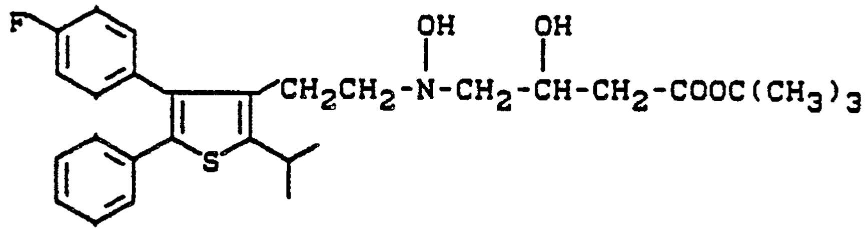

Die Verbindung erhält man analog Beispiel 5 - 7 aus 4-(4-Fluorphenyl)-3-formyl-2-isopropyl-5-phenyl-thiophen.
Farbloser Schaum.
$^1$H-NMR ($CDCl_3$): $\delta$ = 1,38 (d, 6H, isopropyl-$CH_3$); 1,45 (s, 9H, tert.butyl); 2,35 (d, 2H, $CH_2$); 2,5 - 2,65 (m, 4H); 2,75 (m, 2H, $CH_2$); 3,35 (m, 2H, isopropyl-H + OH); 4,15 (m, 1H, HO-$\underline{CH}$); 5,1 (b, 1H, OH); 7,0 - 7,2 (m, 9H, Aromaten-H).

Beispiel 29

N-{2-[4-(4-Fluorphenyl)-2-isopropyl-5-phenyl-thiophen-3-yl]-ethyl}-N-(3-carboxy-2-hydroxy-propyl)-hydroxylamin-hydrobromid

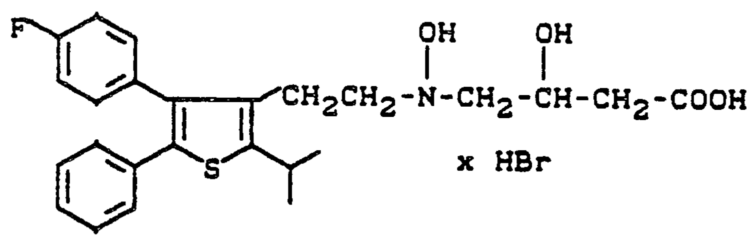

0,7 g (1,4 mmol) der Verbindung aus Beispiel 28 wird in 25 ml 1 n Bromwasserstoffsäure und 5 ml Dioxan 1 h am Rückfluß gekocht. Man zieht die Lösemittel im Vakuum ab und kristallisiert aus Ether um.
Ausbeute: 0,64 g (84%) farblose Kristalle
Schmp.: 135 - 140 °C (Zers.)
$R_f$ = 0,4 (Chloroform/Methanol 3:1)
$^1$H-NMR ($CD_3OD$): $\delta$ = 1,4 (d, 6H, isopropyl-$CH_3$); 2,5 (d, 2H, $CH_2$); 3,0 (m, 2H, $CH_2$); 3,15 -3,5 (m, überlagert durch $CD_3OD$-Signal); 4,3 (b, 1H); 7,1 - 7,3 (m, 9H, Aromaten-H).

Beispiel 30

1-(4-Fluorphenyl)-4-methyl-2-phenyl-penten-3-on

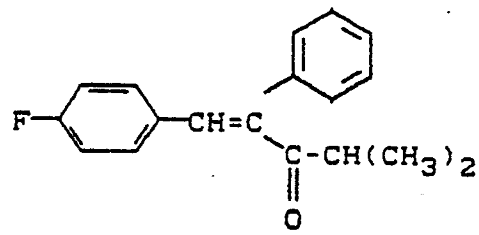

31 g (0,25 mol) 4-Fluorbenzaldehyd, 40,6 g (0,25 mol) Benzylisopropyl-keton, 2,5 ml Piperidin und 1,75 ml Eisessig werden in 200 ml Toluol 20 h am Wasserabscheider gekocht. Nach Abziehen des Lösemittels wird über eine 20 cm Vigreux-Kolonne destilliert.
Ausbeute: 56,8 g (85%) gelbliches Öl
Sdp.: 140 °C (0,2 mbar), das im Kühlschrank durchkristallisiert (Schmp.: 47 °C)

Beispiel 31

3-(4-Fluorphenyl)-5-methyl-1-nitro-3-phenyl-hexan-4-on

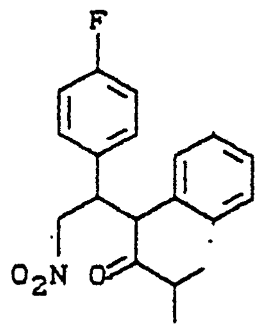

Zu einer Lösung von 53 g (0,2 mol) Beispiel 30 und 29 g (0,47 mol) Nitromethan in 200 ml Actonitril, tropft man bei Raumtemperatur eine Lösung von 31 g (0,2 mol) DBU in 100 ml Acetonitril und rührt über Nacht bei Raumtemperatur. Das Lösemittel wird verdampft, der Rückstand zwischen 500 ml 1 N Salzsäure und 500 ml Dichlormethan verteilt, die organische Phase mit gesättigter Natriumchlorid- und Natriumhydrogencarbonat-Lösung gewaschen und über Natriumsulfat getrocknet. Den Rückstand kristallisiert man aus Ether um.
Ausbeute: 26,4 g (40%) farblose Kristalle
Schmp.: 178 °C
$^1$H-NMR ($CDCl_3$): $\delta$ = 0,68 + 0,77 (zwei d, 6H, isopropyl-$CH_3$); 2,4 (m, 1H, isopropyl-H); 4,15 - 4,5 (m, 4H); 7,0 (t, 2H, F-C-CH); 7,25 - 7,45 (m, 7H, Aromaten-H).

Beispiel 32

4-Fluorphenyl-1,2-diisopropyl-3-phenyl-pyrrol

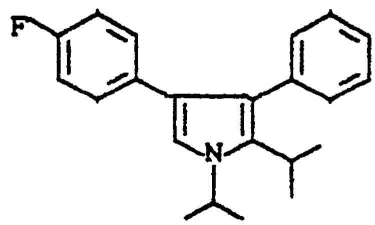

Eine Lösung von 26,5 g (80 mmol) Beispiel 31 in 200 ml Ethanol und 50 ml (0,1 mol) 2 N Natronlauge wird bei 0 °C zu einer Lösung von 22,5 g (0,2 mol) Schwefelsäure in 100 ml Ethanol und 50 ml Wasser getropft und dann 1 h bei Raumtemperatur gerührt. Man filtriert von wenig Niederschlag ab und verteilt das eingeengte Filtrat zwischen Chloroform und Wasser, wäscht die organische Phase mit 1 N Salzsäure und gesättigter Natriumwasserstoffcarbonat-Lösung und trocknet über Natriumsulfat. Nach Abziehen des Lösemittels bleiben 24 g eines dunklen Öls (Rohprodukt des 2-Fluorphenyl-5-methyl-4-oxo-3-phenylhexanals).
$R_f$ = 0,2 (Petrolether/Essigester 2:1).
Obiges Öl wird zusammen mit 29 g (0,5 mol) Isopropylamin in 400 ml Toluol gelöst. Dazu tropft man bei 0 °C 15,3 g (80 mmol) Titantetrachlorid in 50 ml Toluol und rührt 6 h bei Raumtemperatur. Man saugt über Kieselgur ab, wäscht mit Toluol gut nach, extrahiert das Filtrat zweimal mit 6 N Salzsäure, wäscht mit gesättigter Bicarbonatlösung und trocknet über Natriumsulfat. Nach dem Einengen bleiben 26 g eines dunklen Öls, das über eine kurze Kieselgelsäule filtriert wird (Petrolether-Dichlormethan 2:1).
Aus dem Eluat erhält man 3,25 g (13%) farblose Kristalle.
Schmp.: 150 °C
$^1$H-NMR ($CDCl_3$): $\delta$ = 1,25 (d, 6H, C-isopropyl-$CH_3$); 1,5 (d, 6H, N-isopropyl-$CH_3$); 3,2 (sept, 1H, C-isopropyl-H); 4,55 (sept, 1H, N-isopropyl-H); 6,8 (s, 1H, 5-H); 6,8 - 7,3 (m, 9H, Aromaten-H).

## Beispiel 33

3-(4-Fluorphenyl)-2-formyl-1,5-diisopropyl-4-phenyl-pyrrol

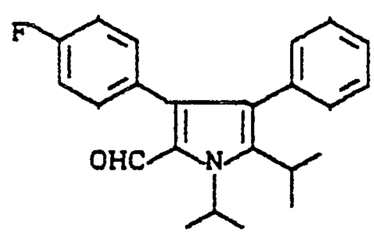

Zu einer Lösung von 3,25 g (10 mmol) Beispiel 32 in 50 ml Dimethylformamid tropft man bei Raumtemperatur 0,92 g (10 mmol) Phosphoroxychlorid, rührt 1 h bei Raumtemperatur und 2 h bei 40 °C und gießt dann in 40 ml eiskalte 1 N Natronlauge (pH ~ 9). Man extrahiert dreimal mit Essigester, wäscht die organische Phase mit Wasser und trocknet über Natriumsulfat. Nach Verdampfen des Lösemittels bleibt ein fester Rückstand (3,5 g), der aus Methylenchlorid / Petrolether umkristallisiert wird.
Ausbeute: 3,0 g (85%) leicht gefärbte Kristalle
Schmp.: 160 °C.

## Beispiel 34

1-[3-(4-Fluorphenyl)-1,5-diisopropyl-4-phenyl-pyrrol-2-yl]-2-nitro-ethen

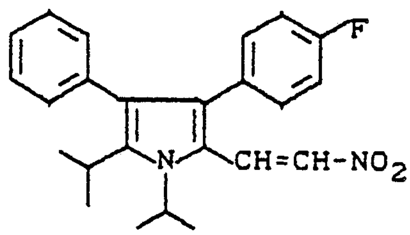

1,1 g (3,15 mmol) Beispiel 33 und 32 mg (0,53 mmol) 1,2-Diaminoethan werden in 12 ml Nitromethan 17 h bei 75 °C gerührt. Man nimmt in Dichlormethan auf, wäscht zweimal mit Wasser, trocknet über Natriumsulfat und kristallisiert den Rückstand aus Ether/Petrolether um.
Ausbeute: 1,09 g (88%) orange Kristalle
Schmp.: 238 °C
$^1$H-NMR ($CDCl_3$): δ = 1,3 (d, 6H, C-isopropyl-$CH_3$); 1,75 (d, 6H, N-isopropyl-H); 3,28 (m, 1H, C-isopropyl-$CH_3$); 4,85 (m, 1H, N-isopropyl-H); 6,6 (db, 1H, Olefin-H); 6,9 - 7,25 (m, 9H, Aromaten-H); 8,85 (db, 1H, Olefin-H).

Beispiel 35

N-{-2-[3-(4-Fluorphenyl)-1,5-diisopropyl-4-phenyl-pyrrol-2-yl]-ethyl}-N-(3-tert.butoxycarbonyl-2-hydroxy-propyl)-hydroxylamin

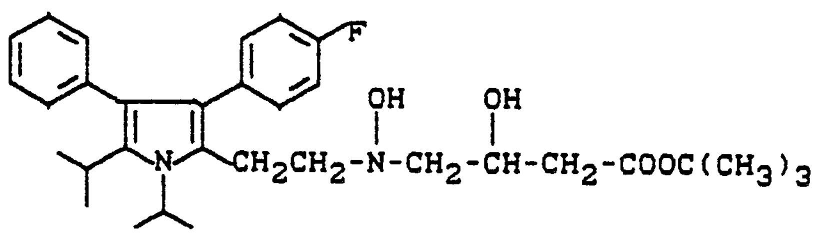

Die Verbindung wird analog Beispiel 6 und 7 aus der Verbindung des Beispiels 34 erhalten.
Farbloser Schaum.
$^1$H-NMR ($CDCl_3$): δ = 1,05 - 1,3 (b, 6H, isopropyl-$CH_3$); 1,45 (s, 9H, tert.butyl); 1,62 (d, 6H, isopropyl-$CH_3$); 2,35 (b, 2H, $CH_2$); 2,45 - 2,75 (b, 4H, $CH_2$); 2,85 (b, 2H, $CH_2$); 3,15 (b, 1H, OH); 3,3 (m, 1H, C-isopropyl-H); 4,15 (b, 1H, HO-CH); 4,6 (b, 1H, N-isopropyl-H); 5,2 (b, 1H, OH); 6,75 - 7,2 (mb, 9H, Aromaten-H).

Beispiel 36

N-{2-[3-(4-Fluorphenyl)-1,5-diisopropyl-4-phenyl-pyrrol-2-yl]-ethyl}-N-(3-carboxy-2-hydroxy-propyl)-hydroxyl-amin-hydrobromid

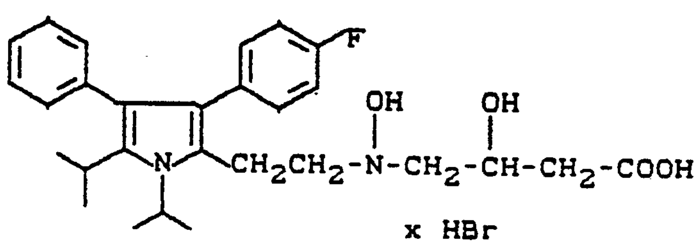

Analog Beispiel 29 aus der Verbindung des Beispiels 35. Das Rohprodukt wird mit je 200 ml Chlorofom und Methanol 10:1 und 5:1 auf der 25-fachen Menge Kieselgel (230-400 mesh) chromatographiert.
Ausbeute: 56% farblose Kristalle (aus Ether), die sich ab 170° C zersetzen.
$R_f$ = 0,45 (Chloroform:Methanol 3:1)
$^1$H-NMR ($CD_3OD$): $\delta$ = 1,05 - 1,3 (b, 6H, isopropyl-$CH_3$); 1,6 (d, 6H, isopropyl-$CH_3$); 2,3 -3,2 (b, 8H); 4,2 (b, 1H, HO-CH); 4,7 (b, 1H, N-isopropyl); 6,7 - 7,2 (b, 9H, Aromaten-H).

## Anwendungsbeispiel

### Beispiel 37

Die Enzymaktivitätsbestimmung wurde modifiziert nach G.C. Ness et al., Archives of Biochemistry and Biophysics 197, 493 - 499 (1979) durchgeführt. Männliche Ricoratten (Körpergewicht 300 - 400 g) wurden 11 Tage mit Altrominpulverfutter, dem 40 g Colestyramin/kg Futter zugesetzt war, behandelt. Nach Dekapitation wurde den Tieren die Leber entnommen und auf Eis gegeben. Die Lebern wurden zerkleinert und in einem Homogenisator 3 mal in 3 Volumen 0,1 m Saccharose, 0,05 m KCl, 0,04 m $K_xH_y$ Phosphat (Mischung aus $K_2HPO_4$ und $KH_2PO_4$ mit pH 7,2), 0,03 m Ethylendiamintetraessigsäure, 0,002 m Dithiothreit (SPE)-Puffer (Saccharose-Phosphat-Ethylendiamintetraessigsäure-Puffer) pH 7,2, homogenisiert. Anschließend wurde 15 Minuten zentrifugiert und das Sediment verworfen. Der Überstand wurde 75 Minuten sedimentiert. Das Pellet wird in 1/4 Volumen SPE-Puffer aufgenommen, nochmals homogenisiert und anschließend erneut 60 Minuten zentrifugiert. Das Pellet wird mit der 5-fachen Menge ihres Volumes SPE-Puffer aufgenommen, homogenisiert und bei -78° C eingefroren und gelagert (= Enzymlösung).

Zur Testung wurden die Testverbindungen (bzw. Mevinolin als Referenzsubstanz) in Dimethylformamid unter Zugabe von 5 Vol.-% 1 n NaOH gelöst und mit 10 µl in verschiedenen Konzentrationen in den Enzymtest eingesetzt. Der Test wurde nach 20 Minuten Vorinkubation der Verbindun gen mit dem Enzym bei 37° C gestartet. Der Testansatz betrug 0,380 ml und enthielt 4 µMol Glucose-6-Phosphat, 1,1 mg Rinderserumalbumin, 2,1 µMol Dithiothreit, 0,35 µMol NADP, 1 Einheit Glucose-6-Phosphatdehydrogenase 35 µMol $KxH_y$-Phosphat PH 7,2, 20 µl Enzympräparation und 56 nMol 3-Hydroxy-3-methyl-glutaryl Coenzym A (Glutaryl-3-$^{14}$C) 100 000 dpm.

Man inkubierte 60 Minuten bei 37° C und stoppte die Reaktion durch Zusatz von 300 µl 0,24 m HCl ab. Nach einer Nachinkubation von 60 Minuten bei 37° C wurde der Ansatz zentrifugiert und 600 µl des Überstandes auf eine 0,7 x 4 cm mit 5-Chlorid Anionenaustauscher mit einer Korngröße 100-200 mesh (Anionenaustauscher) gefüllte Säule aufgetragen. Es wurde mit 2 ml dest. Wasser nachgewaschen und Durchlauf plus Waschwasser mit 3 ml einer Scintillationsflüssigkeit versetzt und im Scintillationszähler gezählt. $IC_{50}$-Werte wurden durch Auftrag der prozentualen Hemmung gegen die Konzentration der Verbindung im Test durch Intrapolation bestimmt. Zur Bestimmung der relativen inhibitorischen Potenz wurde der $IC_{50}$-Wert der Referenzsubstanz Mevinolin als 100 gesetzt und mit dem simultan bestimmten $IC_{50}$-Wert der Testverbindung verglichen.

## Ansprüche

1. Substituierte Hydroxylamine der allgemeinen Formel

$$R^1\text{-}CH_2CH_2\text{-}\overset{OH}{\overset{|}{N}}\text{-}CH_2\text{-}\overset{OH}{\overset{|}{C}}H\text{-}CH_2\text{-}COOR \qquad (I)$$

in welcher
R - für Wasserstoff,
- für einen Esterrest, oder
- für ein Kation steht,
und
$R^1$ - für eine Gruppe der Formel

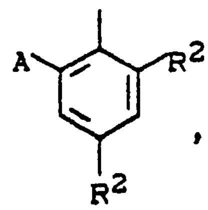 , 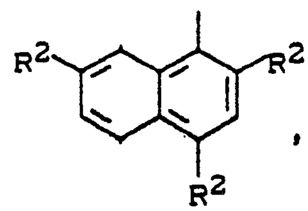 , 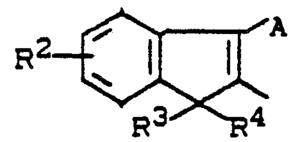 ,

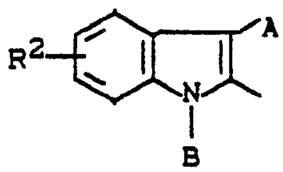 , 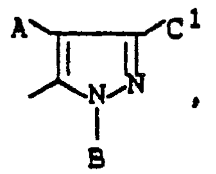 , 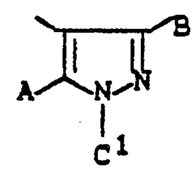 ,

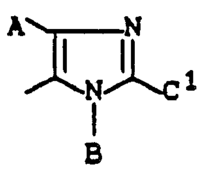 , 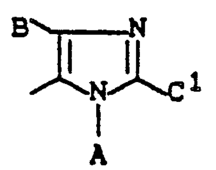 , 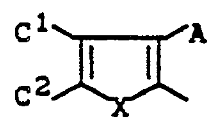 ,

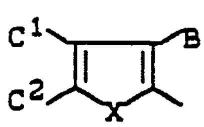 , 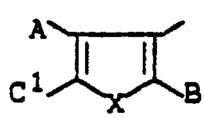 oder 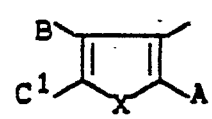

steht,
worin
$R^2$ - Wasserstoff, Halogen oder geradkettiges oder verzweigtes Alkyl mit bis zu 6 Kohlenstoffatomen bedeutet,
$R^3$, $R^4$ - gleich oder verschieden sind und
- Wasserstoff, Halogen oder geradkettiges oder verzweigtes Alkyl mit bis zu 6 Kohlenstoffatomen bedeuten,
oder
$R^3$ und $R^4$ gemeinsam eine Tetramethylenkette bilden,
A - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluoromethyl, Trifluormethoxy, Trifluormethylthio, Alkoxycarbonyl oder durch eine Gruppe der Formel $-NR^5R^6$ substituiert sein kann,
worin
$R^5$, $R^6$ - gleich oder verschieden sind und Al kyl, Aryl, Aralkyl, Acyl, Alkylsulfonyl oder Arylsulfonyl bedeuten,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,
worin
$R^5$ und $R^6$ die oben angegebene Bedeutung haben,
B - Cycloalkyl bedeutet, oder - Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,
worin
$R^5$, $R^6$ - gleich oder verschieden sind und Alkyl, Aryl, Aralkyl, Acyl, Alkylsulfonyl oder Arylsulfonyl bedeuten,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
X - O, S oder N-$C^3$ bedeutet,
und
$C^1$, $C^2$, $C^3$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -$NR^5R^6$,
worin
$R^5$, $R^6$ die oben angegebene Bedeutung haben,
oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio, oder Aralkylsulfonyl, wobei die Heteroaryl- und Arylreste bis zu 3-fach gleich oder verschieden durch Halogen, Cyano, Trifluormethyl, Trifluormethoxy, Alkyl, Alkoxy, Alkylthio, oder Alkylsulfonyl substituiert sein kann,
oder
- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -$NR^5R^6$ substituiert sein kann,
worin
$R^5$ und $R^6$ die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -$NR^5R^6$,
worin
$R^5$ und $R^6$ die oben angegebene Bedeutung haben.

2. Substituierte Hydroxylamine nach Anspruch 1, wobei
R - für Wasserstoff oder
- für einen physiologisch verträglichen Esterrest steht, oder
- für ein physiologisch verträgliches Kation steht,
und
$R^1$ - für eine Gruppe der Formel

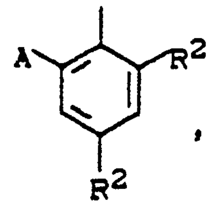 , 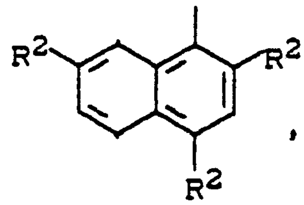 , 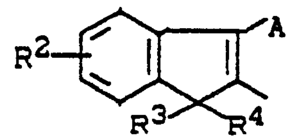 ,

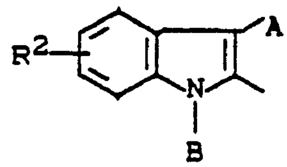 , 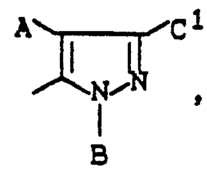 , 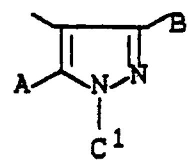 ,

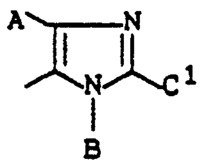 , 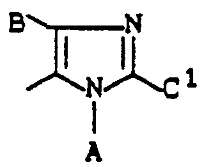 , 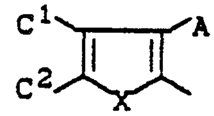 ,

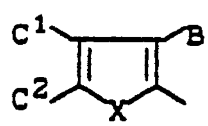 , 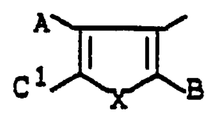 oder 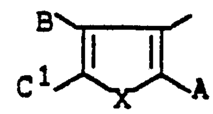

steht,
worin
$R^2$ - Wasserstoff, Fluor, Chlor, Brom, oder
- geradkettiges oder verzweigtes Alkyl mit bis zu 5 Kohlenstoffatomen bedeutet,
$R^3$, $R^4$ - gleich oder verschieden sind und
- Wasserstoff, Fluor, Chlor, Brom, oder
- geradkettiges oder verzweigtes Alkyl mit bis zu 4 Kohlenstoffatomen bedeuten,
oder
$R^3$ und $R^4$ gemeinsam eine Tetramethylenkette bilden,
A - Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,
wobei
$R^5$ und $R^6$ gleich oder verschieden sind und Niederalkyl, Phenyl, Benzyl, Acetyl, Benzoyl, Phenylsulfonyl oder Niederalkylsulfonyl bedeuten,
B - Cyclopropyl, Cyclopentyl oder Cyclohexyl bedeutet, oder
- Niederalkyl bedeutet, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,
worin
$R^5$ und $R^6$ die oben angegebene Bedeutung haben,
oder durch Pyridyl, Pyrimidyl, Pyrazinyl, Pyridazinyl, Chinolyl, Isochinolyl, Pyrryl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

X - O, S oder N-$C^3$ bedeutet,

und

$C^1$, $C^2$, $C^3$ gleich oder verschieden sind und

- Wasserstoff, oder
- Cyclopropyl, Cyclopentyl oder Cyclohexyl bedeuten, oder
- Niederalkyl bedeuten, das substituiert sein kann durch Fluor, Chlor, Brom, Cyano, Niederalkoxy, Niederalkylthio, Niederalkylsulfonyl, Trifluormethyl, Trifluormethoxy, Trifluormethylsulfonyl, Niederalkoxycarbonyl, Benzoyl, Niederalkylcarbonyl, durch eine Gruppe der Formel $-NR^5R^6$,

worin

$R^5$ und $R^6$ die oben angegebene Bedeutung haben,

oder durch Pyridyl, Pyrimidyl, Pyrazinyl, Pyridazinyl, Chinolyl, Isochinolyl, Pyrrolyl, Indolyl, Thienyl, Furyl, Imidazolyl, Oxazolyl, Thiazolyl, Phenyl, Phenoxy, Phenylthio, Phenylsulfonyl, Benzyloxy, Benzylthio, Benzylsulfonyl, Phenylethoxy, Phenylethylthio oder Phenylethylsulfonyl, wobei die genannten Heteroaryl- und Arylreste bis zu 2-fach gleich oder verschieden durch Fluor, Chlor, Brom, Niederalkyl, Niederalkoxy, Trifluormethyl, oder Trifluormethoxy substituiert sein können,

- Thienyl, Furyl, Thiazolyl, Isothiazolyl, Oxazolyl, Isoxazolyl, Pyridyl, Pyrimidyl, Pyrazinyl, Pyridazinyl, Indolyl, Isoindolyl, Chinolyl, Isochinolyl, Phthalazinyl, Chinoxalinyl, Chinazolinyl, Cinnolinyl, Benzothiazolyl, Benzoxazolyl oder Benzimidazolyl bedeutet, das bis zu 2-fach gleich oder verschieden substituiert sein kann durch Fluor, Chlor, Brom, Niederalkyl, Niederalkoxy, Phenyl, Phenoxy, Trifluormethyl, Trifluormethoxy oder Niederalkoxycarbonyl, oder
- Phenyl oder Naphthyl bedeutet, das bis zu 4-fach gleich oder verschieden substituiert sein kann durch Niederalkyl, Niederalkoxy, Niederalkylthio, Niederalkylsulfonyl, Phenyl, Phenyloxy, Phenylthio, Phenylsulfonyl, Benzyl, Benzyloxy, Benzylthio, Benzylsulfonyl, Phenethyl, Phenylethoxy, Phenylethylthio, Phenylethylsulfonyl, Fluor, Chlor, Brom, Cyano, Trifluormethyl, Trifluormethoxy, Trifluormethylthio, Niederalkoxycarbonyl oder durch eine Gruppe der Formel $-NR^5R^6$,

wobei

$R^5$ und $R^6$ die oben angegebene Bedeutung haben.

3. Substituierte Hydroxylamine nach den Ansprüchen 1 und 2, wobei

R - für Wasserstoff, Methyl, Ethyl, Propyl, Isopropyl, Butyl, Isobutyl, tert.Butyl oder Benzyl, oder

- für ein Magnesium- oder Ammoniumkation steht,

und

$R^1$ - für eine Gruppe der Formel

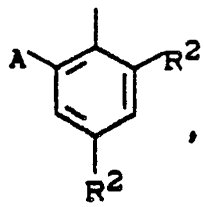,
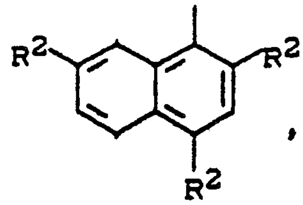,
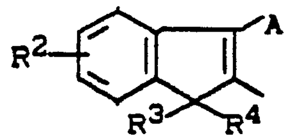,

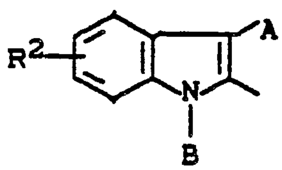,
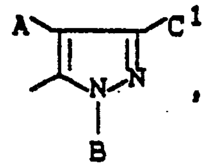,
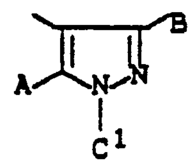,

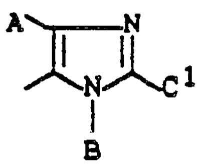,
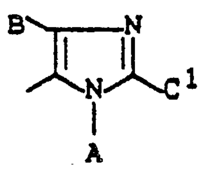,
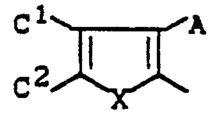,

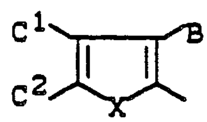,
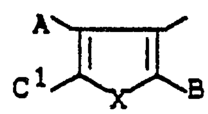
oder
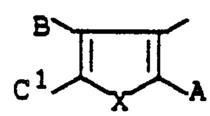

steht,
worin
$R^2$ - Wasserstoff, Fluor, Chlor oder
- geradkettiges oder verzweigtes Alkyl mit bis zu 4 Kohlenstoffatomen bedeutet,
$R^3$, $R^4$ - gleichzeitig Wasserstoff, Fluor, Chlor oder
- Alkyl mit bis zu 3 Kohlenstoffatomen bedeuten,
oder
$R^3$ und $R^4$ gemeinsam eine Tetramethylenkette bilden,
A - Pyridyl, Pyrimidyl, Chinolyl oder Isochinolyl bedeutet, das durch Fluor, Chlor, Methyl, Methoxy oder Trifluormethyl substituiert sein kann, oder
- Phenyl bedeutet, das bis zu 3-fach gleich oder verschieden substituiert sein kann durch Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Methylthio, Ethylthio, Propylthio, Isopropylthio, Methylsulfonyl, Ethylsulfonyl, Propylsulfonyl, Isopropylsulfonyl, Phenyl, Phenoxy, Benzyl, Benzyloxy, Fluor, Chlor, Brom, Cyano, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl oder tert.Butoxycarbonyl,
B - Cyclopropyl, Cyclopentyl oder Cyclohexyl bedeutet, oder
- Methyl, Ethyl, Propyl, Isopropyl, Butyl, sec.-Butyl oder tert.Butyl bedeutet, das substi tuiert sein kann durch Fluor, Chlor, Brom, Cyano, Methoxy, Ethoxy, Propoxy, Isopropoxy, Butoxy, sec.Butoxy, tert.Butoxy, Methylthio, Ethylthio, Propylthio, Isopropylthio, Methylsulfonyl, Ethylsulfonyl, Propylsulfonyl, Isopropylsulfonyl, Trifluormethyl, Trifluormethoxy, Methoxycarbonyl, Ethoxycarbonyl, Butoxycarbonyl, Isobutoxycarbonyl, tert.-Butoxycarbonyl, Benzoyl, Acetyl, Pyridyl, Pyrimidyl, Thienyl, Furyl, Phenyl, Phenoxy, Phenylthio, Phenylsulfonyl, Benzyloxy, Benzylthio oder Benzylsulfonyl,
X - O, S oder N-$C^3$ bedeutet,
$C^1$, $C^2$, $R^3$ gleich oder verschieden sind und
- Wasserstoff, Cyclopropyl, Cyclopentyl oder Cyclohexyl bedeuten, oder
- Methyl, Ethyl, Propyl, Isopropyl, Butyl, Isobutyl, tert.Butyl, Pentyl, Isopentyl, Hexyl oder Isohexyl bedeuten, das substituiert sein kann durch Fluor, Chlor, Brom, Cyano, Methoxy, Ethoxy, Propoxy, Isopropoxy, Butoxy, Isobutoxy, tert.Butoxy, Methylthio, Ethylthio, Propylthio, Isopropylthio, Butylthio, Isobutylthio, tert.Butylthio, Methylsulfonyl, Ethylsulfonyl, Propylsulfonyl, Isopropylsulfonyl, Butylsulfonyl, Isobutylsulfonyl,

tert.Butylsulfonyl, Trifluormethyl, Trifluormethoxy, Methoxycarbonyl, Ethoxycarbonyl, Propoxycarbonyl, Isopropoxycarbonyl, Butoxycarbonyl, Isobutoxycarbonyl, tert.Butoxycarbonyl, Benzoyl, Acetyl, Ethylcarbonyl, oder durch eine Gruppe $-NR^5R^6$,
wobei
$R^5$ und $R^6$ gleich oder verschieden sind und Methyl, Ethyl, Propyl, Isopropyl, Butyl, Isobutyl, tert.Butyl, Phenyl, Benzyl, Acetyl, Methylsulfonyl, Ethylsulfonyl, Propylsulfonyl, Isopropylsulfonyl oder Phenylsulfonyl bedeuten,
oder durch Pyridyl, Pyrimidyl, Pyrazinyl, Pyridazinyl, Chinolyl, Isochinolyl, Thienyl, Furyl, Phenyl, Phenoxy, Phenylthio, Phenylsulfonyl, Benzyloxy, Benzylthio oder Benzylsulfonyl, wobei die genannten Heteroaryl- und Arylreste durch Fluor, Chlor, Methyl, Ethyl, Propyl, Isopropyl, Isobutyl, tert. Butyl, Methoxy, Ethoxy, Propoxy, Isopropoxy, Butoxy, Isobutoxy, tert.-Butoxy, Trifluormethyl oder Trifluormethoxy substituiert sein können, oder
- Thienyl, Furyl, Pyridyl, Pyrimidyl, Pyrazinyl, Pyridazinyl, Oxazolyl, Isooxazolyl, Imidazolyl, Pyrazolyl, Thiazolyl, Isothiazolyl, Chinolyl, Isochinolyl, Benzoxazolyl, Benzimidazolyl oder Benzthiazolyl bedeutet, wobei die genannten Reste durch Fluor, Chlor, Methyl, Ethyl, Propyl, Isopropyl, Butyl, Isobutyl, tert.Butyl, Methoxy, Ethoxy, Propoxy, Isopropoxy, Butoxy, Isobutoxy, tert.Butoxy, Phenyl, Phenoxy, Trifluormethyl, Trifluormethoxy, Methoxycarbonyl, Ethoxycarbonyl, Isopropoxycarbonyl, Propoxycarbonyl, Butoxycarbonyl, Isobutoxycarbonyl oder tert.Butoxycarbonyl substituiert sein können, oder
- Phenyl bedeutet, das bis zu 3-fach gleich oder verschieden durch Methyl, Ethyl, Propyl, Isopropyl, Butyl, Isobutyl, tert.Butyl, Pentyl, Isopentyl, Hexyl, Isohexyl, Methoxy, Ethoxy, Propoxy, Isopropoxy, Butoxy, Isobutoxy, tert.-Butoxy, Methylthio, Ethylthio, Propylthio, Isopropylthio, Butylthio, Isobutylthio, tert.-Butylthio, Methylsulfonyl, Ethylsulfonyl, Propylsulfonyl, Isopropylsulfonyl, Butylsulfonyl, Isobutylsulfonyl, tert.Butylsulfonyl, Phenyl, Phenoxy, Phenylthio, Phenylsulfonyl, Benzyl, Benzyloxy, Benzylthio, Benzylsulfonyl, Fluor, Chlor, Brom, Cyano, Trifluormethyl, Trifluormethoxy, Trifluormethylthio, Methoxycarbonyl, Ethoxycarbonyl, Propoxycarbonyl, Isopropoxycarbonyl, Butoxycarbonyl, Isobutoxycarbonyl, tert.Butoxycarbonyl oder durch eine Gruppe $-NR^5R^6$ substituiert sein kann,
wobei
$R^5$ und $R^6$ die oben angegebene Bedeutung haben.

4. Substituierte Hydroxylamine der allgemeinen Formel

$$R^1\text{-}CH_2CH_2\text{-}\ \overset{\displaystyle OH}{\overset{|}{N}}\ \text{-}CH_2\text{-}\ \overset{\displaystyle OH}{\overset{|}{C}}H\ \text{-}CH_2\text{-}COOR \qquad (I)$$

in welcher
R und $R^1$ die in Anspruch 1 angegebene Bedeutung haben
zur therapeutischen Behandlung.

5. Verfahren zur Herstellung von substituierten Hydroxylaminen der allgemeinen Formel

$$R^1\text{-}CH_2CH_2\text{-}\ \overset{\displaystyle OH}{\overset{|}{N}}\ \text{-}\ CH_2\text{-}\ \overset{\displaystyle OH}{\overset{|}{C}}H\ \text{-}CH_2\text{-}COOR \qquad (I)$$

in welcher
R - für Wasserstoff,
- für einen physiologisch verträglichen Esterrest, oder
- für ein physiologisch verträgliches Kation steht,
und
$R^1$ - für eine Gruppe der Formel

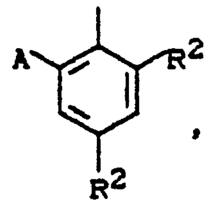,
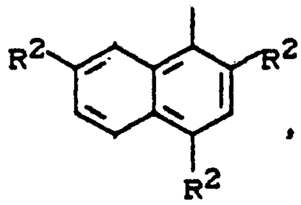,
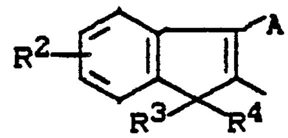,

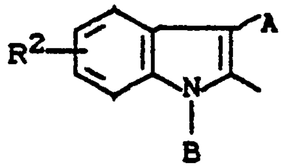,
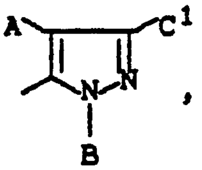,
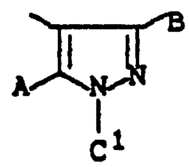,

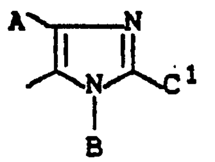,
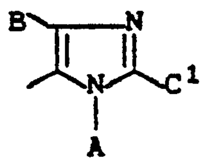,
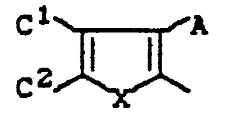,

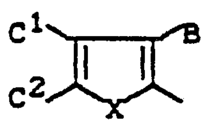,
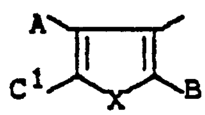 oder
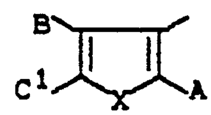

steht,

worin

$R^2$ - Wasserstoff, Halogen oder geradkettiges oder verzweigtes Alkyl mit bis zu 6 Kohlen stoffatomen bedeutet,

$R^3$, $R^4$ - gleich oder verschieden sind und

- Wasserstoff, Halogen oder geradkettiges oder verzweigtes Alkyl mit bis zu 6 Kohlenstoffatomen bedeuten,

oder

$R^3$ und $R^4$ gemeinsam eine Tetramethylenkette bilden,

A - Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel $-NR^5R^6$ substituiert sein kann,

worin

$R^5$,$R^6$ - gleich oder verschieden sind und Alkyl, Aryl, Aralkyl, Acyl, Alkylsulfonyl oder Arylsulfonyl bedeuten,

oder

- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel $-NR^5R^6$,

worin

$R^5$ und $R^6$ die oben angegebene Bedeutung haben,

B - Cycloalkyl bedeutet, oder

- Alkyl bedeutet, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel $-NR^5R^6$,

worin

$R^5$, $R^6$ - gleich oder verschieden sind und Alkyl, Aryl, Aralkyl, Acyl, Alkylsulfonyl oder Arylsulfonyl bedeuten,

oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein können,
X - O, S oder N-$C^3$ bedeutet,
und
$C^1$, $C^2$, $C^3$ gleich oder verschieden sind und
- Wasserstoff bedeuten, oder
- Cycloalkyl bedeuten, oder
- Alkyl bedeuten, das substituiert sein kann durch Halogen, Cyano, Alkoxy, Alkylthio, Alkylsulfonyl, Trifluormethyl, Trifluormethoxy, Trifluormethylthio, Trifluormethylsulfonyl, Alkoxycarbonyl, Acyl oder durch eine Gruppe der Formel -$NR^5R^6$,
worin
$R^5$ und $R^6$ die oben angegebene Bedeutung haben, oder durch Carbamoyl, Dialkylcarbamoyl, Sulfamoyl, Dialkylsulfamoyl, Heteroaryl, Aryl, Aryloxy, Arylthio, Arylsulfonyl, Aralkoxy, Aralkylthio oder Aralkylsulfonyl, wobei die Heteroaryl- und Arylreste der letztgenannten Substituenten bis zu 3-fach gleich oder verschieden durch Halogen, Cyano, Trifluormethyl, Trifluormethoxy, Alkyl, Alkoxy, Alkylthio oder Alkylsulfonyl substituiert sein kann,
oder
- Heteroaryl bedeutet, das bis zu 3-fach gleich oder verschieden durch Halogen, Alkyl, Alkoxy, Alkylthio, Alkylsulfonyl, Aryl, Aryloxy, Arylthio, Arylsulfonyl, Trifluormethyl, Trifluormethoxy, Trifluormethylthio, Alkoxycarbonyl oder durch eine Gruppe der Formel -$NR^5R^6$ substituiert sein kann,
worin
$R^5$ und $R^6$ die oben angegebene Bedeutung haben,
oder
- Aryl bedeutet, das bis zu 5-fach gleich oder verschieden substituiert sein kann durch Alkyl, Alkoxy, Alkylthio, Alkylsulfonyl, Aryl, Aryloxy, Arylthio, Arylsulfonyl, Aralkyl, Aralkoxy, Aralkylthio, Aralkylsulfonyl, Halogen, Cyano, Nitro, Trifluormethyl, Trifluormethoxy, Trifluormethylthio, Alkoxycarbonyl, Sulfamoyl, Dialkylsulfamoyl, Carbamoyl, Dialkylcarbamoyl oder durch eine Gruppe der Formel -$NR^5R^6$,
worin
$R^5$ und $R^6$ die oben angegebene Bedeutung haben,
dadurch gekennzeichnet, daß man
Hydroxylamine der allgemeinen Formel (II)
$R^1$-$CH_2CH_2$-NH-OH (II)
in welcher
$R^1$ die oben angegebene Bedeutung hat,
mit Epoxiden der allgemeinen Formel (III)

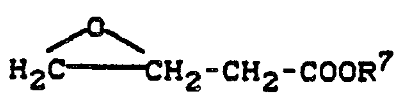 (III)

in welcher
$R^7$ - für geradkettiges oder verzweigtes Alkyl mit bis zu 6 Kohlenstoffatomen steht,
in inerten Lösemitteln umsetzt,
und dann im Fall der Herstellung der Säure, die Ester verseift,
und im Fall der Herstellung der Salze die Säuren mit den entsprechenden Basen umsetzt.

6. Verfahren nach Anspruch 5, dadurch gekennzeichnet, daß die Umsetzung im Temperaturbereich von 0 bis 150 °C durchgeführt wird.

7. Hydroxylamine der allgemeinen Formel
$R^1$-$CH_2CH_2$-NH-OH (II)
in welcher
$R^1$ die in Anspruch 1 angegebene Bedeutung hat.

8. Verfahren zur Herstellung von Hydroxylaminen der allgemeinen Formel
$R^1$-$CH_2CH_2$-NH-OH (II)
in welcher
$R^1$ die in Anspruch 5 angegebene Bedeutung hat,
dadurch gekennzeichnet, daß man
in einem ersten Schritt Aldehyde der allgemeinen Formel (IV)
R-CHO (IV)

in welcher
R - für Wasserstoff,
- für einen Esterrest, oder
- für ein Kation steht,
mit Nitromethan in inerten Lösemitteln, gegebenenfalls in Anwesenheit von Hilfsmitteln, umsetzt, und anschließend in einem zweiten Schritt die erhaltenen Nitroverbindungen der allgemeinen Formel (V)
$R-CH=CH-NO_2$ (V)
in inerten Lösemitteln gegebenenfalls in Anwesenheit eines Hilfsmittels reduziert.

9. Verfahren nach Anspruch 8, dadurch gekennzeichnet, daß es im Temperaturbereich von 0 bis 150 °C durchgeführt wird.

10. Arzneimittel, enthaltend substituierte Hydroxylamine der allgemeinen Formel

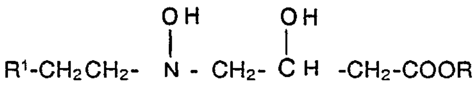
(I)

in welcher
R und $R^1$ die in Anspruch 1 angegebene Bedeutung haben.

11. Arzneimittel nach Anspruch 10, enthaltend 0,5 bis 98 Gew.-% der substituierten Hydroxylamine.

12. Verwendung von substituierten Hydroxylaminen der Formel

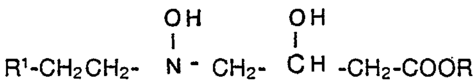
(I)

in welcher
R und $R^1$ die in Anspruch 1 angegebene Bedeutung haben
zur therapeutischen Behandlung.

13. Verwendung von substituierten Hydroxylaminen der Formel

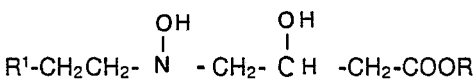
(I)

in welcher
R und $R^1$ die in Anspruch 1 angegebene Bedeutung haben,
zur Herstellung von Arzneimitteln.

14. Verwendung nach Anspruch 13 zur Herstellung von Inhibitoren der 3-Hydroxy-3-methyl-glutaryl-Coenzym A-Reduktase und von Inhibitoren der Cholesterolbiosynthese.